# EUROPEAN PATENT SPECIFICATION

(11) **EP 0 514 540 B1**
(45) Date of publication and mention of the grant of the patent: **17.07.1996**
(21) Application number: 90913731.7
(22) Date of filing: 19.09.1990
(51) Int. Cl.: C07D 487/04, C07D 519/00, A61K 31/505, A61K 31/535, A61K 31/54, A61K 31/55

(54) **PYRROLO 2,3-d]PYRIMIDINE DERIVATIVE, PROCESS FOR PREPARING THE SAME, AND PHARMACEUTICAL PREPARATION COMPRISING THE DERIVATIVE AS ACTIVE INGREDIENT**
PYRROLO[2,3-D]PYRIMIDINDERIVATE, VERFAHREN ZUR HERSTELLUNG UND ARZNEIMITTELZUSAMMENSETZUNGEN DIE DIESE ENTHALTEN
DERIVE DE PYRROLO 2,3-d]PYRIMIDINE, SON PROCEDE DE PREPARATION, ET PREPARATION PHARMACEUTIQUE COMPRENANT LE DERIVE COMME INGREDIENT ACTIF

(30) Priority: 19.09.1989 JP 242933/89
(43) Date of publication of application: 25.11.1992
(73) Proprietor: TEIJIN LIMITED, Osaka-shi Osaka 541 (JP)
(72) Inventor: SAKUMA, Yasuji, Hino-shi, Tokyo 191 (JP); HASEGAWA, Masaichi, Hino-shi, Tokyo 191 (JP); KATAOKA, Kenichiro, Setagaya-ku, Tokyo 156 (JP); HOSHINA, Kenji, Hino-shi, Tokyo 191 (JP); YAMAZAKI, Noboru, Hachioji-shi, Tokyo 192-01 (JP); KADOTA, Takashi, Hachioji-shi, Tokyo 193 (JP); YAMAGUCHI, Hisao, Hino-shi, Tokyo 191 (JP)
(74) Representative: Votier, Sidney David
(86) International application number: JP9001200
(87) International publication number: WO9104254

(56) References cited:
- JP-A-63 275 598
- J. Heterocyclic Chem., 25 (6), p. 1893-1898 (1988), KANDASAMY RAMASAMY et al. "A Facile and Improved Synthesis of Tubercidin and Certain Related Pyrrolo 2,3-d pyrimidine Nucleosides by the Stereospecific Sodium Salt Glycosylation Procedure (I)".
- J. Heterocyclic Chem., 24 (2), p. 425-30 (1987), KURT EGER et al. "Selected Reactions on the 0-Aminonitrile System of Substituted Pyrroles (I)".

## Description

### TECHNICAL FIELD

The present invention relates to a novel pyrrolo[2,3-d]pyrimidine derivative, a process for producing same, and a pharmaceutical preparation comprising the derivative, and more particularly, relates to a novel pyrrolo[2,3-d]pyrimidine derivative having independently a substituted or unsubstituted amino group at the 2- and 4- positions of the pyrimidine ring, a pharmaceutically acceptable acid addition salt thereof, a process for producing same, and a pharmaceutical preparation comprising same as an active ingredient; particularly, a pharmaceutical preparation useful for the treatment, i.e., prophylaxis and therapy, of hypoxemia associated with respiratory diseases.

### BACKGROUND ART

Compounds having the pyrrolo[2,3-d]pyrimidine skeleton of the formula: 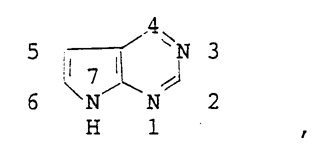
are known to have various important pharmacological actions. For example, it is known that antibacterial compounds are substituted by amino groups at both the 2- and 4- positions of the skeleton (see, U.K. Patent 812, 366, and Townsend L.B. et al, J. Med. Chem., Vol. 31, 1501(1988), etc.). and that compounds useful as a herbicide and antibiotic have a primary amino group as the amino group (Okuda et al, Nippon Noyaku-gakkaishi, Vol. 6, 9(1981), Pedersen E.B. et al., Chemica Scripta, Vol. 28, 201(1988), etc.), Other known antiviral compounds have an amino group at the 2- and 4- positions as well as a sugar residue at the 7- position of the skeleton (e.g., E.P. Publication No. 57548).

Nevertheless, in particular, pyrrolo[2,3-d]pyrimidine derivatives having an alkyl or alkenyl group at the 7- position, an amino group substituted by alkyl or alkenyl group at the 2- position, and a cyclic amino or chain substituted amino group at the 4- position, have not been described in the prior art.

### DISCLOSURE OF INVENTION

The present inventors made extensive and intensive research into pyrrolo[2,3-d]pyrimidine derivatives and a process for producing same, and as a result, surprisingly found that, of the compounds not disclosed in the prior art, those of the formula [I] described below are particularly, efficacious for the prophylaxis and therapy of hypoxemia associated with respiratory diseases. Note, in the treatment of hypoxemia, to this day it has not been known that a compound can suffice in the light of both the pharmaceutical effect and toxicity there.

Thus, in accordance with the present invention there are provided, pyrrolo[2,3-d]pyrimidine derivatives having the formula [I] described below, pharmaceutically acceptable acid addition salts thereof, a process for producing same, as well as pharmaceutical preparations containing same derivatives or salts as an active ingredient: 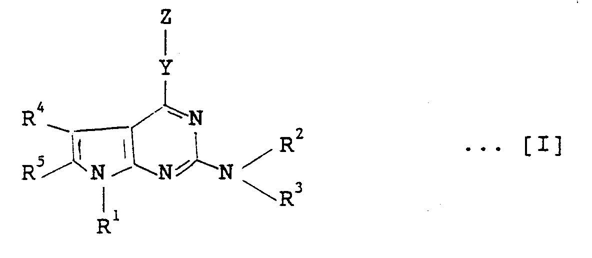
wherein
R¹ represents a hydrogen atom, or an unsubstituted or substituted alkyl, alkenyl or aralkyl group;
R² and R³, independently of each other, represent a hydrogen atom, or an unsubstituted or substituted alkyl, alkenly, aralkyl or alkylcarbonyl group; or R² and R³ are optionally taken together with the adjacent nitrogen atom to form a cyclic amino group;
R⁴ and R⁵ independently of each other, represent a hydrogen atom, halogen atom, or an unsubstituted or substituted alkyl group;
Y is a linking group bonded to the pyrimidine ring via a nitrogen atom therein of the formula 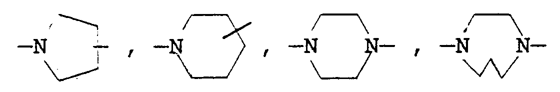
or -N(G)-, wherein G represents a hydrogen atom or an alkyl group;
Z represents a group bonded to a carbon or nitrogen atom in the linking group, and is a hydrogen atom, an unsubstituted or substituted alkyl, alkenyl, aralkyl, aryl, alkylcarbonyl, arylcarbonyl or arakylcarbonyl group; or represents a group bonded to a carbon atom in the linking group, and is a carboxyl, hydroxyl; or an unsubstituted or substituted alkyloxycarbonyl, alkylcarbonyloxy, arylcarbonyloxy, aralkylcarbonyloxy, alkyloxy, alkyloxyiminoalkyl group, or mono- or disubstituted alkyl- and/or alkylcarbonyamino group; or Y and Z are taken together to form morpholino or thio-morpholino group;
each substituent in said substituted group is substituted at a chain or cyclic moiety of the alkyl, alkenyl, aralkyl or aryl moiety, respectively, and represents an alkyl, halogenated alkyl, alkylcarbonyl, alkyloxy, alkylcarbonyloxy, hydroxyl, mono- or di-alkylamino, amino, morpholino, piperidino, nitro or cyano group, or a halogen atom;
with a proviso that R² and R³ do not represent a hydrogen atom at the same time, and that, when R¹ represents hydrogen atom, the combinations wherein one of R² and R³ represents a hydrogen atom and another represents an alkyl group are excluded.

### BEST MODE OF CARRYING OUT THE INVENTION

This invention is disclosed in detail below.

An alkyl moiety of each group, unless defined otherwise, is herein intended to mean a C₁ - C₁₀ straight or branched chain aliphatic hydrocarbon residue, alicyclic hydrocarbon residue or chainaliphatic-alicyclic hydrocarbon residue, and is for example, methyl, ethyl, propyl, isopropyl, butyl, isobutyl, sec-butyl, tert-butyl, pentyl, hexyl, cyclopropyl, cyclobutyl, cyclopentyl, cyclohexyl, cyclopropyl-methyl, cyclobuthymethyl and the like; preferably a C₁ - C₆ lower alkyl group.

The term "alkenyl group" is intended to mean a C₂ - C₆ straight or branched chain aliphatic hydrocarbon residue containing one double bond, for example, allyl, 1-methylallyl, 2-methylallyl, 2-butenyl, 2-methyl-2-butenyl, 3-methyl-2-buteny, 3-butenyl, 2-pentenyl, 3-methyl-2-pentenyl, 2-hexenyl, 3-cyclopropylally, 3-cyclopentenyl, 3-cyclohexenyl, and the like.

The term "aryl group" is intended to mean aromatic hydrocarbon cyclic groups or aromatic heterocyclic groups constructed of a mono-ring or fused ring and examples thereof include phenyl, 1-naphthyl, 2-naphthyl, 2-pyrrolyl, 2-furyl, 2-thienyl, 2-pyridyl and the like.

The term "aralkyl group" is lower aralky groups constructed of lower alkyl groups and arly groups, and containing in total 6 to 20 carbon atoms, for example, benzyl, 1-phenylethyl, 1-methyl-1-phenylethyl, 2-phenylethyl, 3-phenylpropyl, cinnamyl, diphenylmethyl (benzhydryl), triphenylmethyl, 1-naphthylmethyl, 1-(1-naphthy)ethyl, 1,2,3,4-tetrahydronaphtharen-1-yl, 2-pyrrolylmethyl, 2-furfuryl, 2-thienylmethyl and the like.

The above-described alkyl, alkenyl, aryl and aralkyl groups may have a substituent in the chain moiety or cyclic moiety thereof, and examples of the substituent include alkyl groups having about 1 to 4 carbon atoms, halogenated alkyl, alkylcarbonyl, alkyloxy, alkylcarbonyloxy, hydroxyl, mono- or di- substituted alkylamino, amino, morpholino, piperidino, nitro and cyano groups, and halogen atoms, i.e., fluorine, chlorine, bromine or iodine atom, etc.. The groups defined below also may have the above-described substituents in the chain portion or cyclic portion thereof.

In the present invention, the term "alkylcarbonyl group", which is constructed of said lower alkyl and carbonyl group, is intended to mean C₂-C₇ lower alkylacyl groups, such as acetyl, propanoyl, butanoyl, 2-methylpropanoyl, pentanoyl, 2-methylbutanoyl, 3-methylbutanoyl, pivaloyl, hexanoyl, cyclopropylcarbonyl, and the like.

The term "arylcarbonly group" which is constructed of said aryl and carbonyl group, is intended to mean, for example, benzoyl,toluoyl, naphthoyl, 2-pyrrolecarbonyl, 2-furancarbonyl, 2-thiophenecarbonyl, and the like.

The term "aralkylcarbonyl group", which is constructed of said aralkyl and carbonyl group, is intended to mean C₇-C₁₉ aralkylcarbonyl groups, such as phenylacetyl, 3-phenylpropanoyl, 4-phenylbutanoyl, cinnamoyl, diphenylacetyl, naphthylacetyl, 2-pyrrolylacetyl, 2-furylacetyl, 2-thienylacetyl, and the like.

The term "alkyloxycarbonyl group", which is a carboxylic acid ester residue containing said alkyl group, is intended to mean C₂-C₇ lower alkyloxycarbonyl groups, such as methoxycarbonyl, ethoxycarbonyl, propoxycarbonyl, isopropyloxycarbonyl, butoxycarbonyl, tert-butyloxycarbonyl, cyclohexyloxycarbonyl, and the like.

The term "alkylcarbonyloxy group" which is constructed of said alkylcarbonyl and oxy group, is intended to mean C₂-C₇ lower alkylcarbonyloxy groups, such as acetoxy, propanoyloxy, butanoyloxy, 2-methylpropanoyloxy, pentanoyloxy, pivaloyloxy, hexanoyloxy, and the like.

The term "arylcarbonyloxy group", which is constructed of said arylcarbonyl and oxy group, is intended to mean, for example, benzoyloxy, toluoyloxy, naphthoyloxy, 2-pyrrolecarbonyloxy, 2-furancarbonyloxy, 2-thiophenecarbonyloxy, and the like.

The term "aralkylcarbonyloxy group", which is constructed of said aralkylcarbonyl and oxy group, is intended to mean lower aralkylacyloxy groups, such as phenylacetoxy, 3-phenylpropanoyloxy, 4-phenylbutanoyloxy, cinnamoyloxy, 2-pyrrolylacetoxy, 2-furylacetoxy, 2-thienylacetoxy, and the like.

The term "alkyloxy group", which is constructed of said alkyl and oxy group, is intended to mean a C₁-C₆ lower alkyloxy group; such as methoxy, ethoxy, propoxy, butoxy, isopropyloxy, sec-butyloxy, tert-butyloxy, pentyloxy, cyclopropyloxy, cyclopropylmethyloxy, cyclohexyloxy, and the like.

The term "alkyloxyiminoalkyl group" represents a group that, in the alkylcarbonyl groups, the oxygen atom is replaced with a group N-O-alkyl, and has the formula:

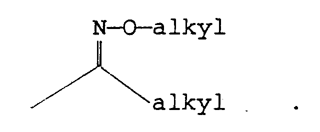

The term "mono- or disubstituted alkyl- and/or alkylcarbonyl-amino group", represents amino groups substituted by one or two substituents selected from the same or different said lower alkyl or lower alkylcarbonyl group is intended to mean a C₁-C₁₀ alkyl- and/or alkylcarbonyl-amino group, such as, methylamino, ethylamino, propylamino, butylamino, cyclohexylamino, dimethylamino, diethylamino, N-methylbuthylamino, acetylamino, propanoylamino, pivaloylamino, N-methylacetylamino and N-ethylacetylamino group, etc..

The term "halogen atom" is intended to mean a fluorine atom, a chlorine atom, a bromine atom and an iodine atom.

According to the above-described definitions, the R¹ in the general formula [I] includes a hydrogen atom, or an unsubstituted or substituted alkyl group, alkenyl group, aralkyl group and alkylacyl group.

Suitable specific examples of the alkyl group include methyl, ethyl, propyl, isopropyl, butyl, cyclopropyl, cyclopropylmethyl, trifluoromethyl, methoxymethyl, methoxyethoxymethyl, aminoethyl, and the like. Suitable specific examples of the alkenyl group include allyl, 2-methylallyl, 2-butenyl, 3-methyl-2-butenyl, 2-fluoroallyl, 3-fluoroally, 2-(trifluoromethyl)allyl, 3-butenyl, and the like. Suitable specific examples of the aralkyl group include benzyl, 4-fluorobenzyl, 4-chlorobenzyl, 3-(trifluoromethyl)benzyl, 4-methoxybenzyl, 2-phenylethyl, 2-[(2-trifluoromethyl)phenyl]ethyl, triphenylmethyl, (4-methoxyphenyl)diphenylmethyl, 2-thienylmethyl, and the like. Suitable specific examples of the alkylacyl group include acetyl, trifluoroacetyl, propanoyl, 2-methylpropanoyl, butanoyl, and the like.

The R² and R³ in the general formula [I] are respectively identical with the R¹, and suitable examples thereof as well are identical with those of R¹. The R² and R³ may be taken together with an adjacent nitrogen atom to form a cyclic amino group. The cyclic amino group is a saturated five- to seven-membered ring and may further have at least one cyclic hetero atom (for example, N, O or S) in its ring other than the above-described nitrogen. Suitable specific examples of the cyclic amino group include 1-pyrrolidinyl, piperidino, 1-piperazinyl, 4-[bis(4-fluorophenyl)methyl]-1-piperazinyl, morpholino, thiomorpholino, 1-perhydro[1,4]diazepinyl, and the like.

The R⁴ and R⁵ in the general formula [I] each independently represent a hydrogen atom, a halogen atom or an substituted or substituted alkyl group. Suitable specific examples of the halogen atom include a fluorine atom, a chlorine atom and a bromine atom. Suitable examples of the alkyl group include methyl, ethyl and trifluoromethyl.

In the general formula [I], the Y represents a linking group, which is bonded to the pyrimidine ring via a nitrogen atom therein, of the formula 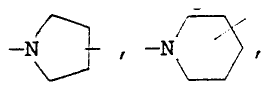 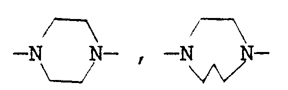
or -N(G)- , wherein G represents a hydrogen atom or an alkyl group. Suitable specific examples of the same inculde groups of the formula: 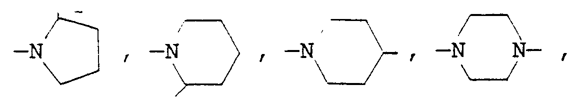
-NH- , -N(CH₃)- and -N(C₂H₅)- .

In the general formula [I], where Z bonds with a carbon atom or nitrogen atom in the linking group, suitable specific examples there of include a hydrogen atom; an alkyl group such as methyl, ethyl, propyl, isopropyl, butyl, cyclohexyl, dimethyaminoethyl, morpholinoethyl, piperidinoethyl, and the like; an alkenyl group such as allyl, 2-methylallyl, 2-butenyl, 3-methyl-2-butenyl, 2-fluoroally, 3-flouroallyl, 2-(trifluoromethyl)allyl, 3-butenyl, and the like; an aralkyl group such as benzyl, 4-fluorobenzyl, 4-chlorobenzyl, 3-(trifluoromethyl)-benzyl, 4-methoxybenzyl, 1-phenylethyl, 1-methyl-1-phenylethyl, 2-phenylethyl, 2-(4-fluorophenyl)-ethyl, 2-(4-chlorophenyl)ethyl, 2-(4-methoxyphenyl)ethyl, 2-[2-(trifluoromethyl)phenyl]-ethyl, cinnamyl, diphenylmethy, bis(4-fluorophenyl)methyl, 1-(1-naphthyl)ethyl, 1,2,3,4-tetrahydronaphthalene-1-yl, 2-pyrrolyl-methyl, 2-furfuryl, 2-thienylmethy, and the like; an aryl group such as phenyl, 4-fluorophenyl, 4-chlorophenyl, 4-methoxphenyl, and the like; an alkylcarbonyl group such as acetyl, trifluoroacetyl, propanoyl, 2-methylpropanoyl, butanoyl, pivaloyl, cyclopropylcarbonyl, and the like; an arylcarbonyl group such as benzoyl, 4-fluorobenzoyl, 4-chlorobenzoyl, 3-methoxybenzoyl, 4-toluoyl, 1-naphthoyl, 1-pyrrolocarbonyl, 2-furylcarbonyl, 2-thiophenecarbonyl, and the like; and an aralkylcarbonyl group such as phenylacetyl, 4-fluorophenylacetyl, 3-phenylpropanonyl, cinnamoyl, diphenylacetyl, and the like.

Where Z bonds with a carbon atom in the linking group, suitable other specific examples include a carboxyl group; hydoxyl group; alkyloxycarbonyl group such as methoxycarbonyl, ethoxycarbonyl, isopropoxycarbonyl and tert-butyloxycarbonyl; alkylcarbonyloxy group such as acetyloxy, propanoyloxy, 2-methylpropanoyloxy and pivaloyloxy; arylcarbonyloxy group such as benzoyloxy, 4-fluorobenzoyloxy, 4-toluoyloxy, 4-chlorobenzoyloxy, 4-methoxybenzoyloxy and 1-naphthoyloxy; aralkylcarbonyloxy group such as phenylacetoxy, 4-fluorophenylacetoxy, 3-phenylpropanoyloxy, 4-phenylbutanoyloxy and cinnamoyloxy; alkyloxy group such as methoxy, ethoxy, propoxy, isopropoxy and butoxy; and an alkyloxyiminoalkyl group such as groups of the formula: 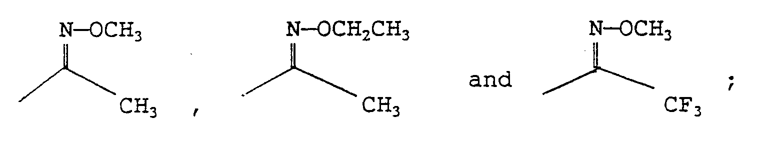
mono- or disubstituted alkyl- and/or alkylcarbonyl-amino group such as methylamino, ethylamino, dimethylamino, diethylamino, acetylamino, pivaloylamino, N-methylacetylamino and N-ethylacetylamino.

Further, preferably Y and Z taken together represent a morpholino group and thiomorpholino group.

Suitable specific examples of pyrrolo[2,3-d]pyrimidine of the general formula [I] in accordance with the present invention include the compounds containing the substituents described in the following table. Note, when the compound has asymmetric carbon atoms in stracture thereof, the compounds of the present invention include all optical isomers.

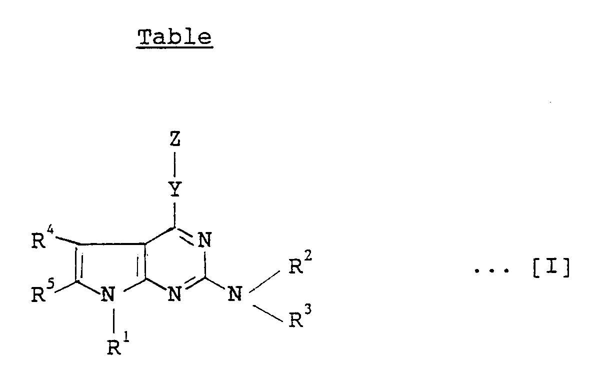

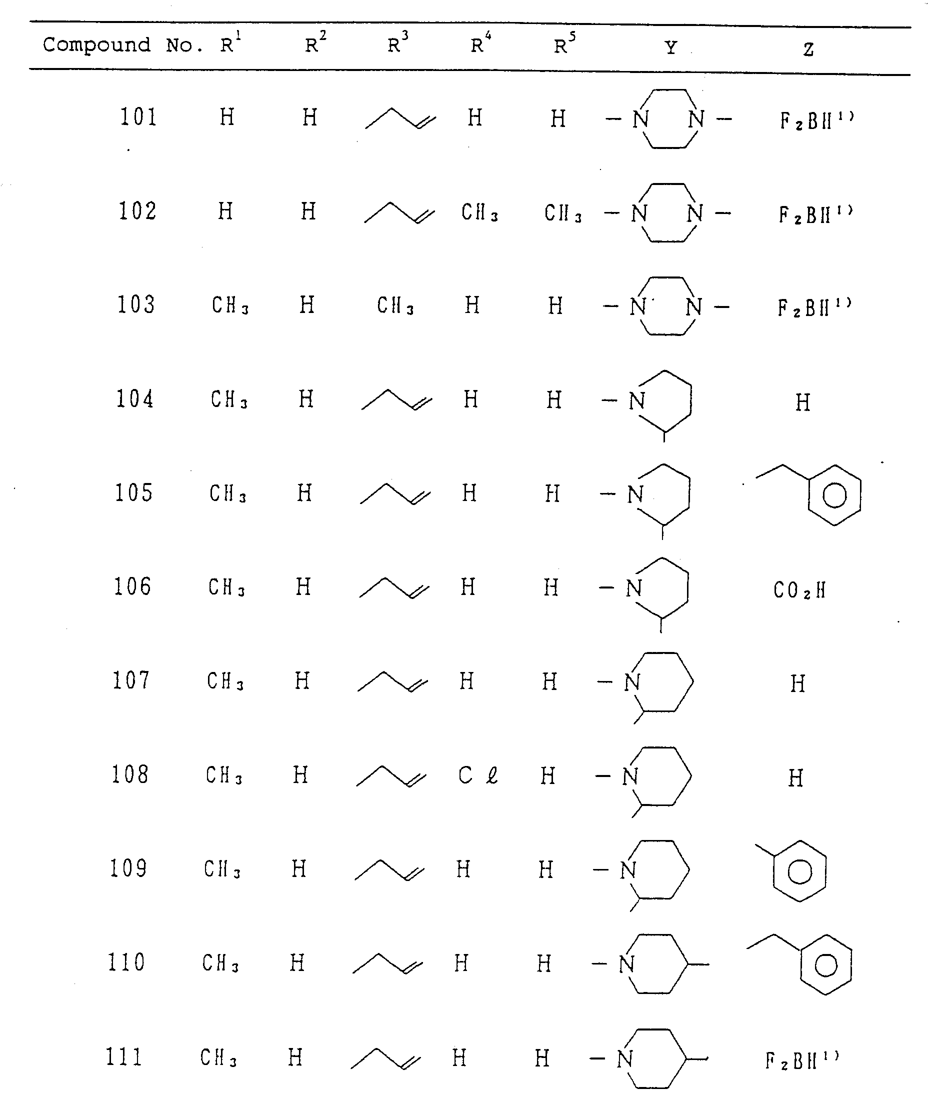

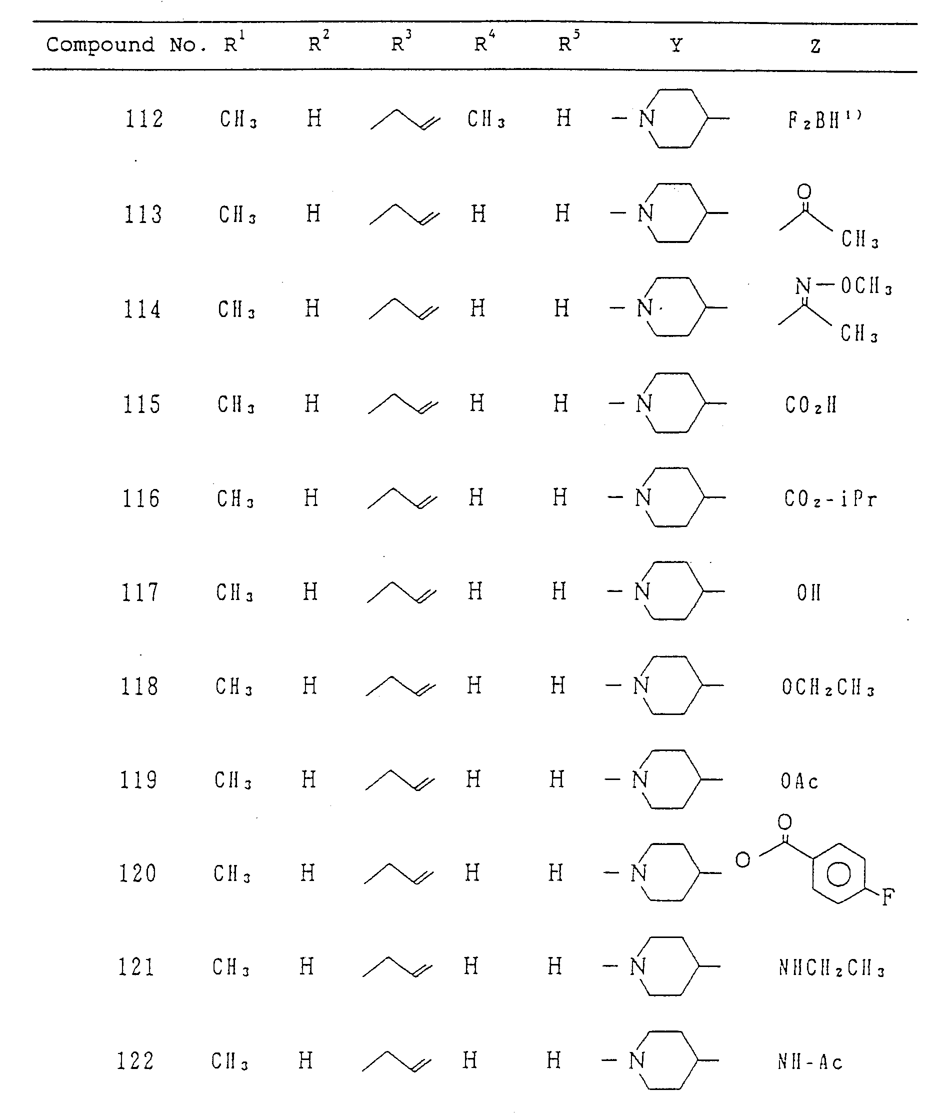

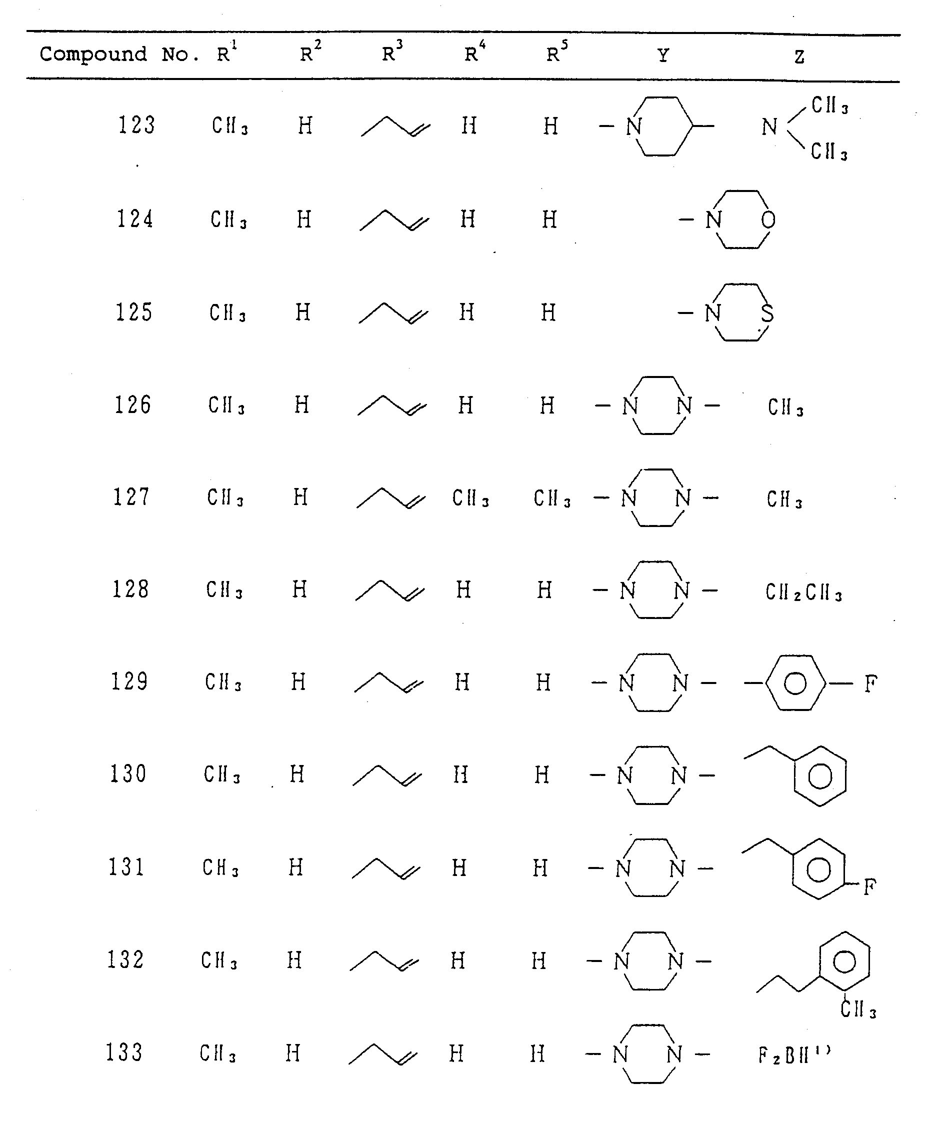

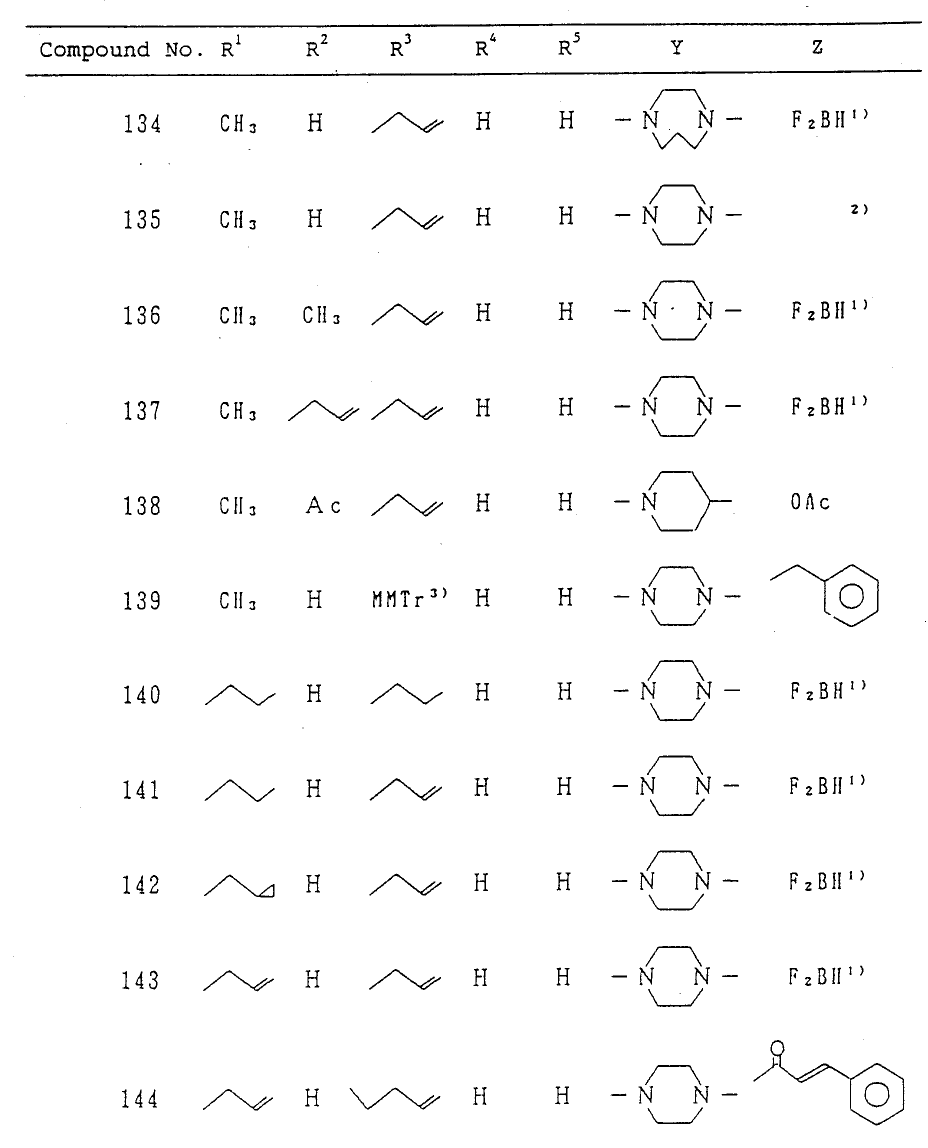

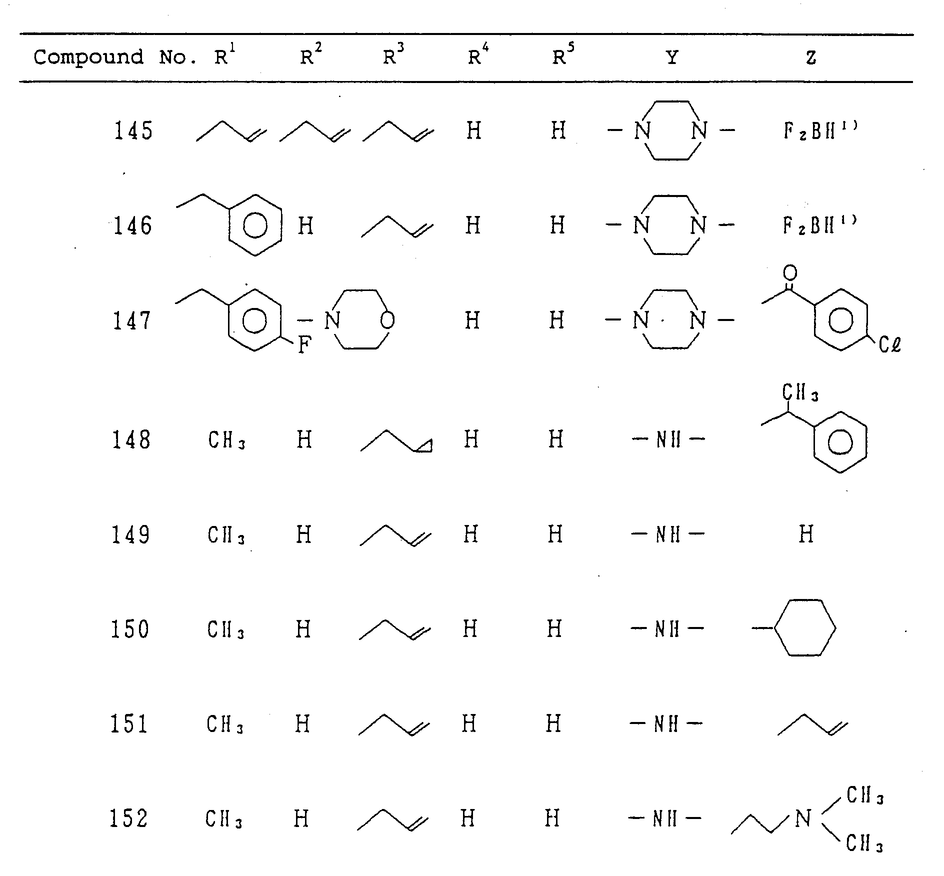

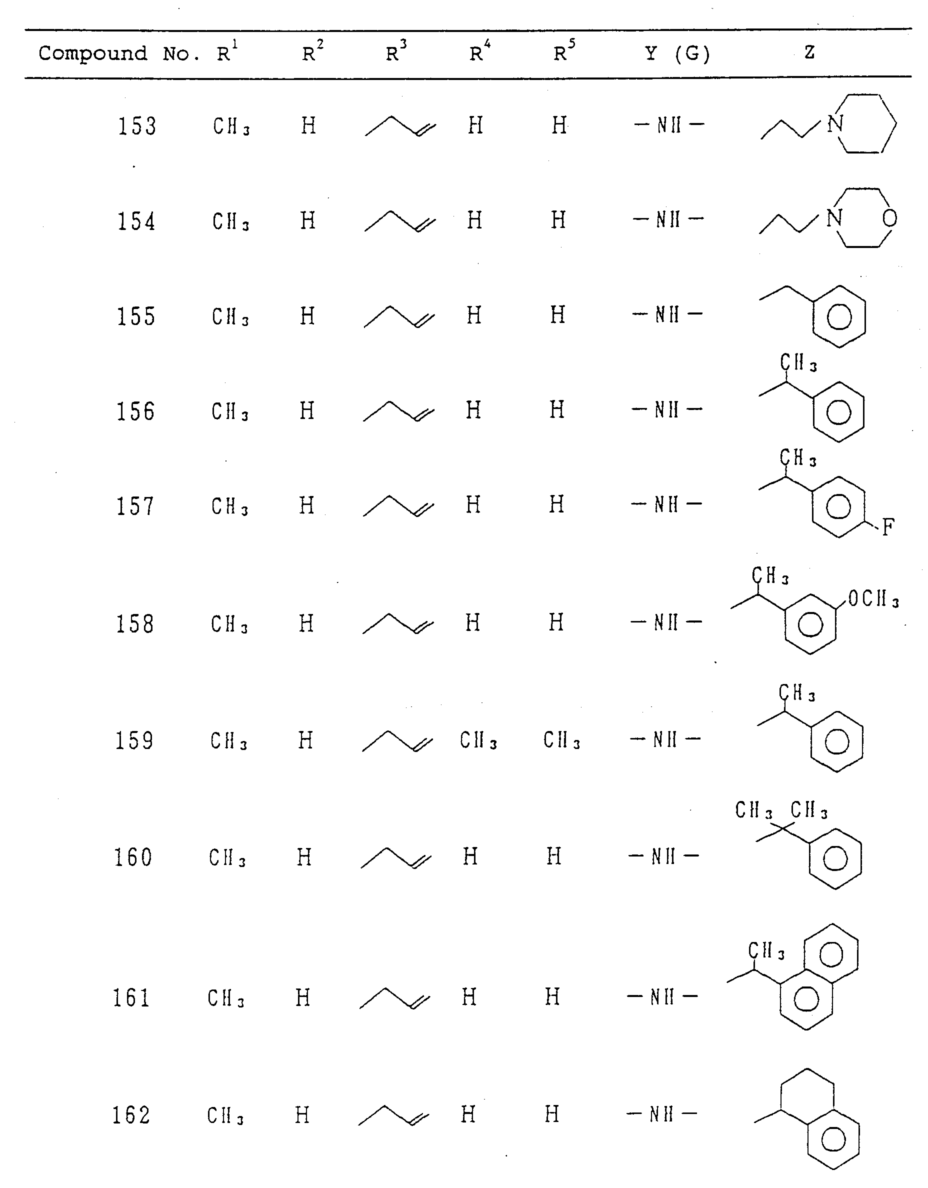

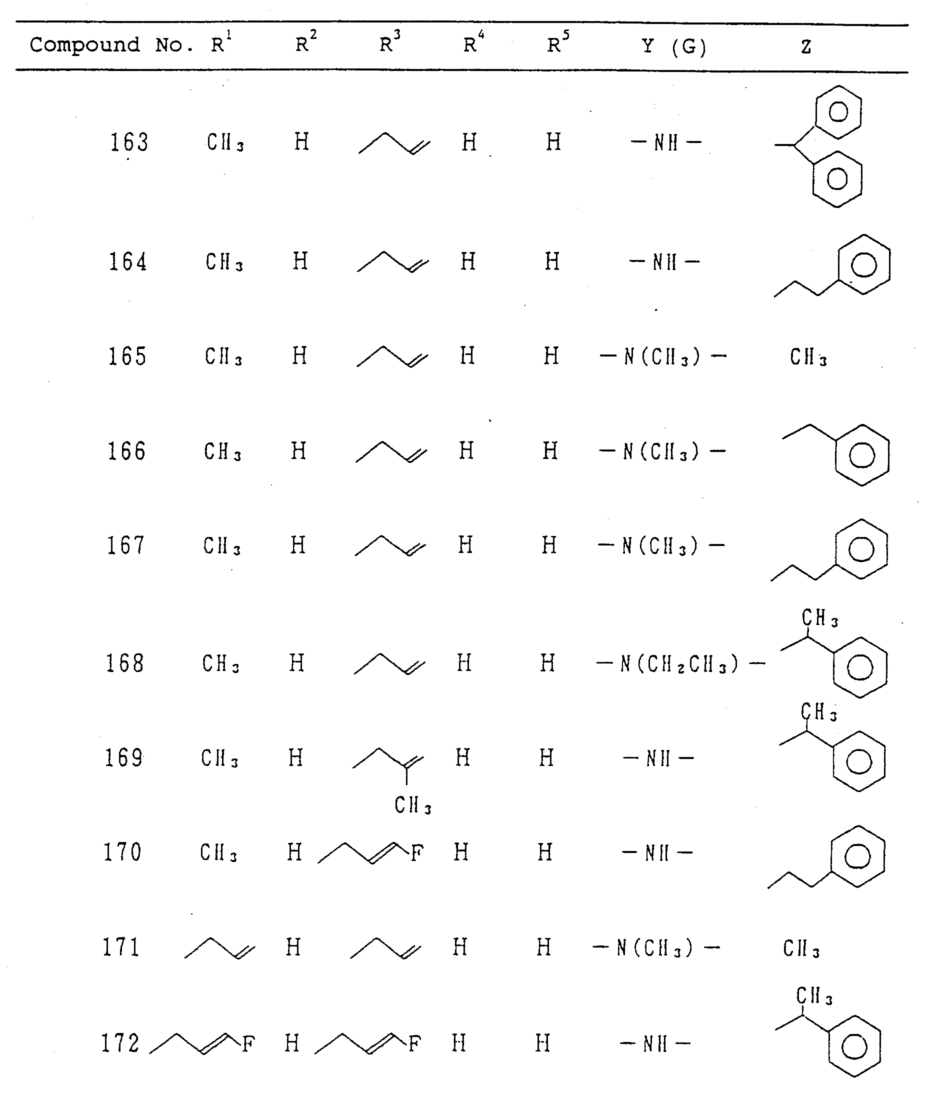

wherein
F₂BH¹⁾, ²⁾ and MMTr³⁾ represent 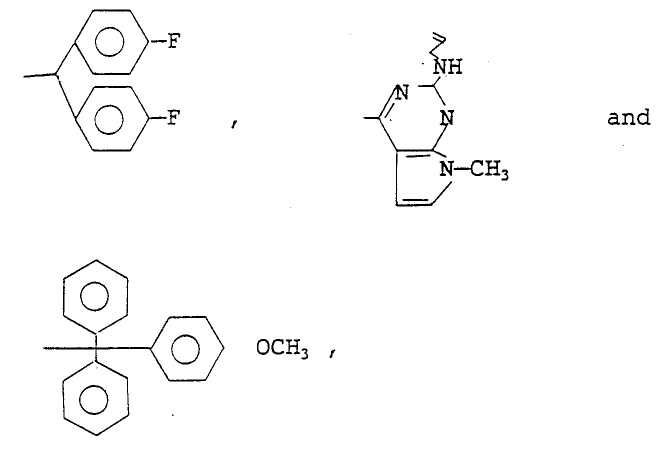
respectively.

The pyrrolo[2,3-d]pyrimidine derivatives in accordance with the present invention may be acid addition salts, and suitable examples of acids forming such salts include inorganic acids such as hydrochloric acid, hydrobromic acid, sulfuric acid, phosphoric acid, nitric acid, boric acid, carbonic acid, and the like; organic carboxylic acids such as formic acid, acetic acid, propionic acid, citric acid, succinic acid, maleic acid, oxalic acid, tartaric acid, maleic acid, fumaric acid, and the like; and organic acids such as methanesulfonic acid, ethanesulfonic acid, benzenesulfonic acid, p-toluene-sulfonic acid, camphorsulfonic acid, and the like.

According to the present invention, a pyrrolo[2,3-d]pyrimidine derivative and pharmaceutically acceptable acid addition salt thereof can be produced by reacting a halogenated pyrrolo[2,3-d]pyrimidine derivative, or acid addition salt, represented by the general formula [II] 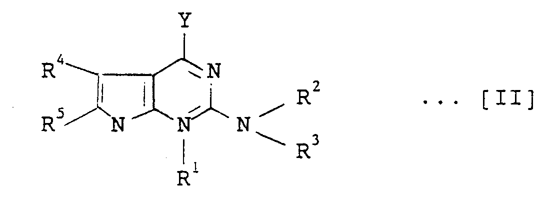
wherein R¹, R², R³, R⁴ and R⁵ have the meanings defined in the formula [I] of claim 1, and X represents a halogn atom with an amine compound represented by the general formula [III]

Z - Y - H [III]

wherein Y and Z have the same meanings as defined in the above formula [I], followed by mixing with an inorganic or organic acid, if necessary.

The halogenated pyrrolo[2,3-d]pyrimidine derivative represented by the formula [II] [wherein R¹ to R⁵ have the meanings defined above in connection with the R¹ to R⁵ in the formula [I] and X represents a halogen atom] may be a novel or known compound. With respect to the known compound, a process for producing same is also known in the art [see, for example, F. Seela et al., Liebigs Ann. Chem., 137 (1983); and M. Legraverend et al., Tetrahedron Lett., Vol. 26, 2001 (1985)].

Also a novel compound can be prepared according to a process of producing a similar known compound. Examples of the halogen atom in the halogenated pyrrolo[2,3-d]pyrimidine derivative include a chlorine atom, a bromine atom and an iodine atom. Such atoms are highly reactive, and a pyrrolo[2,3-d]pyrimidine derivative of interest, represented by the formula [I], can be produced by reacting such atoms with a amine compound represented by the formula [III] wherein Y and Z have the meanings defined above in connection with the Y and Z in the formula [I].

The amine compound represented by the formula [III], as such, is known in the art, or may be produced according to a known process. Suitable specific examples of the above-described amine compound when Y represents a linking group of the formula: 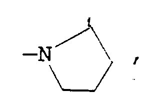
include pyrrolidine, 2-methylpyrrolidine, 2-hydroxymethylpyrrolidine, 2-benzylpyrrolidine, 2-phenylpyrrolidine, 2-carboxypyrrolidine and 2-methoxycarbonylpyrrolidine; when Y represents a linking group of the formula: 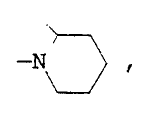
include piperidine, 2-methylpiperidine, 2-hydroxymethylpiperidine, 2-benzylpiperidine, 2-phenylpiperidine, 2-carboxypiperidine and 2-methoxycarbonylpiperidine; when Y represents a linking group of the formula: 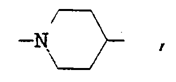
include 4-hydroxypiperidine, 4-methylpiperidine, 4-ethylpiperidine, 4-benzylpiperidine, 4-(4-fluorobenzyl)piperidine, 4-[bis(4-fluorophenyl)methyl]piperidine, 4-phenylpiperidine, 4-acetyloxypiperidine, 4-propanoyloxypiperidine, 4-benzoyloxypiperidine, 4-(4-fluorobenzoyloxy)piperidine, 4-(4-chlorobenzoyloxy)piperidine, 4-(phenylacetyloxy)piperidine, 4-acetylpiperidine, 4-propanoylpiperidine, benzoylpiperidine, 4-(1-methoxyimino)ethylpiperidine, 4-carboxypiperidine, 4-isopropyloxycarbonylpiperidine, 4-methoxypiperidine, 4-ethoxypiperidine, 4-(methylamino)piperidine, 4-(N,N-dimethylamino)-piperidine, 4-(acetylamino)piperidine and 4-(N-methyl-N-acetylamino)piperidine; when Y represents a linking group of the formula: 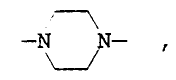
include N-methylpiperazine, N-(2-methoxyethyl)piperazine, N-allylpiperazine, N-phenylpiperazine, N-benzylpiperazine, N-(4-fluorophenyl)piperazine, N-(4-fluorobenzyl)piperazine, N-(2-phenylethyl)piperazine, N-[2-[2-(trifluoromethyl)phenyl]ethyl]piperazine, N-cinnamylpiperazine, N-[bis(4-fluorophenyl)methyl]piperazine, N-acetylpiperazine, N-(4-fluorobenzoyl)piperazine, N-(4-chlorobenzoyl)piperazine, N-(4-fluorophenylacetyl)piperazine and N-cinnamoylpiperazine; and when Y represents a linking group of the formula: 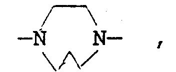
include N-methylperhydro[1,4]diazepine, N-allyl-perhydro[1,4]diazepine, N-benzyl-perhydro[1,4]diazepine, N-(2-phenylethyl) perhydro[1,4]diazepine, N-[bis(4-fluorophenyl)methyl]perhydro[1,4]diazepine and N-acetylperhydro[1,4]diazepine; when Y represents a linking group of the formula: -NH-, include ammonia, allylamine, 2-methylallylamine, methylamine, cyclohexylamine, benzylamine, 4-fluorobenzylamine, 4-chlorobenzylamine, 4-methoxybenzylamine, 1-phenylethylamine, 1-methyl-1-phenylethylamine, 2-phenylethylamine, 2-(4-fluorophenyl)ethylamine, 2-(4-chlorophenyl)ethylamine, 2-(4-methoxyphenyl)ethylamine, 1,1-diphenylmethylamine, 1,1-bis(4-fluorophenyl)methylamine, cinnamylamine, 1-(1-naphthyl)ethylamine, 1,2,3,4-tetrahydronaphthalene-1-yl amine, 2-(N,N-dimethylamino)ethylamine, 2-morpholinoethylamine and 2-piperidinoethylamine; when Y represents a linking group of the formula: -N(CH₃)- or -N(C₂H₅)-, include dimethylamine, diethylamine, N-methylbenzylamine, N-methyl(2-phenylethyl)amine, N-ethylbenzylamine, N-methyl(1-phenylethyl)amine and N-ethyl-(1-phenylethyl)amine; when Y and Z taken together represent a group -Y-Z, include morpholine and thiomorpholine.

The above-described reaction can be conducted, for example, by reacting one equivalent of a halogenated pyrrolo[2,3-d]pyridine derivative represented by the formula [II] with 1 to 30 equivalents of a amine compound represented by the formula [III] in the absence or presence of a solvent. If necessary, a base also may be present in the reaction system, and examples of the base include inorganic bases such as sodium hydroxide, potassium hydroxide, sodium carbonate and potassium carbonate, and organic bases such as triethylamine, diethylaniline, dimethylaniline and pyridine.

The reaction temperature is from -20 to 300°C, preferably from a room temperature to 200°C, and the reaction time is usually 72 hours or less.

Examples of the reaction solvent include halogenated hydrocarbons such as dichloromethane, chloroform, trichloroethane and carbon tetrachloride; aromatic hydrocarbons such as benzene, toluene and xylene; alcohols such as methanol, ethanol, isopropyl alcohol, butanol and tert-butanol; ethers such as diethyl ether, tetrahydrofuran and dioxane; and aprotic polar solvents such as dimethylformamide, dimethylacetamide, dimethylsulfoxide and sulfolane.

After the completion of the reaction, the desired product, a pyrrolo[2,3-d]pyrimidine derivative represented by the formula [1], can be isolated by general separating and purifing procedure, i.e., concentration, extraction, recrystallization, and chromatography, etc. The compound [I] can be also converted to a pharmaceutically acceptable acid addition salt according to a conventional method.

The entire steps of the reaction, including general steps utilized for producing the above-described starting material of the formula [II], are as follows: 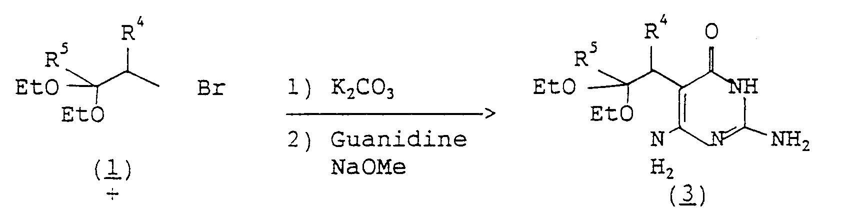 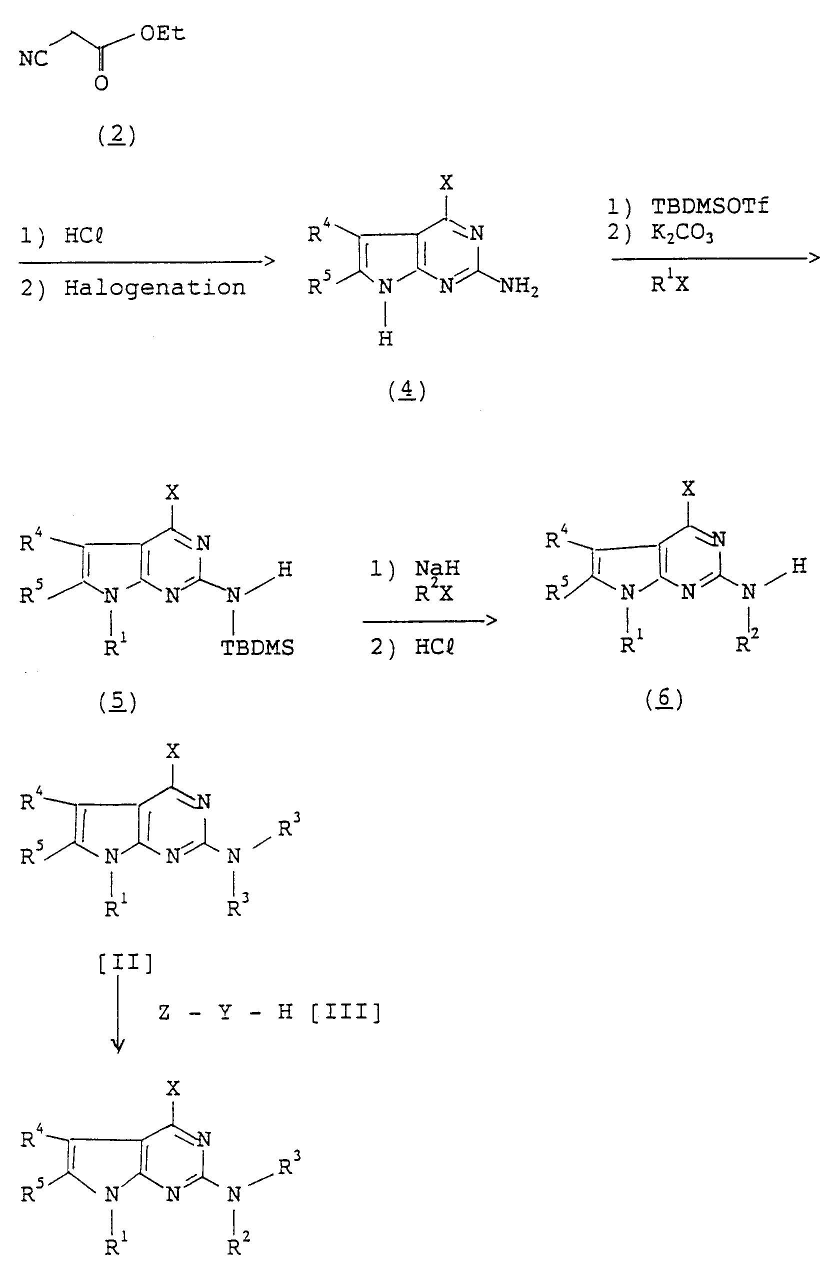
wherein R¹, R², R³, R⁴ and R⁵, as well as X, Y and Z, have the meanings as defined above, and TBDMSOTf represents tert-butyl-dimethyl-silyl-trifluoromethanesulfonate.

The reaction steps for the compound of formula [II] are summarized as follows:
The compound of the formula (3) can be obtained by a reaction of acetal (1) with ethyl cyanoacetate (2) in an alkaline condition, following by a ring-closing reaction using guanidine in the presence of a strong base.

The compound of formula (4) can be obtained by a ring-closing reaction of the resulting compound (3) in the presence of hydrochloric acid, followed by a halogenation according to a usual method, e.g., with phosphorus oxychloride.

The compound of formula (5) can be obtained by a silylation of the resulting compound (4) with TBDMSOTf, followed by a reaction with R¹X, wherein X is a halogen atom, in an alkaline condition.

The compound of formula (6) can be obtained by a reaction of the resulting compound (5) with R⁵X in the presence of a strong base, followed by a desilylation with hydrochloric acid.

The compounds, to which are selectively introduced the substituents R¹, R² and R³, of the formula [II] can be obtained by a reaction of the final compound (6) with R³X, in the presence of a strong base.

The present compounds exhibit an excellent pharmacological action against hypoxemia associated with various respiratory diseases. It is generally known that, in pneumonopathy, e.g., pneumonectasis, bronchitis, bronchial asthma, interstitial preumonia and pneumonophthisis, the partial pressure of oxygen (PaO₂) in arterial blood lowers according as the pathosis is severer or chronic. In this case, symptoms such as a feeling of fatigue, shortness of breath and choking feeling occur, and in a serious state, dyspnea, cyanosis and a disturbance of consciousness occur.

Therefore, a pharmaceutical preparation capable of raising and improving the PaO₂ lowered due to such respiratory diseases has been desired in the art. Further, it is often shown that, in such diseases, the partial pressure of carbon dioxide (PaCO₂) in arterial blood increases conversely to a decrease of PaO₂, and in this case, there is a need for a pharmaceutical preparation that can not only increase PaCO₂ but also decrease PaO₂.

The compounds in accordance with the present invention have actions such that they enhance the respiratory function of the lung, that one mainly increases only PaO₂, and that another increases PaO₂ and decreases PaCO₂, at the same time, and thus the present compounds are useful for the treatment of hypoxemia associated with various respiratory diseases.

The pharmacological effect of the compound in accordance with the present invention can be demonstrated by an acute hypoxemia model using an experimental animal. For example, the acute hypoxemia (having a lower PaO₂ value) model can be prepared by administering intratracheally a fine powder, such as carbon powder, silica gel, glass beads or dental impression material, in a small animal, e.g., rat, to lower the respiratory function [see, for example, Munakata et al., Preprints of the 35th Symposium of Japan Society of Anesthesiology, 179 (1988)]. Also the acute hypoxemia (having a lower PaO₂ value) model can be prepared by administering intratracheally a mucosa-prophiogistic acid, e.g., acetic acid and crotonic acid. Therefore, the compounds in accordance with the present invention were orally or, parenterally administered to the above-described model animal, and the arterial blood was collected after a given period of time and subjected to a measurement of PaO₂ (or PaCO₂) by a blood gas analyzer. As a result, a significant increase of PaO₂ (or decrease of PaCO₂) in comparison with that before the administration, was observed.

The pyrrolo[2,3-d]pyrimidine derivative and its acid addition salt in accordance with the present invention can be administered orally or as a parenteral administration such as an intravenous, subcutaneous, intramuscular, percutaneous, intrarectal or other administration.

Examples of the dosage form for the oral administration include tablets, pills, granules, powders, suspensions and capsules.

The tablets can be formulated by a conventional method through the use of, for example, excipients such as lactose, starch and crystalline cellulose; binders such as carboxymethylcellulose, methylcellulose and polyvinylpyrrolidone; and disintegrators such as sodium alginate, sodium hydrogencarbonate and sodium laurylsulfate.

Similarly, the pills, powders and granules can be formulated by a conventional method through the use of the above-described excipients, etc. The solutions and suspensions can be formulated by a conventional method through the use of, for example, glycerin esters such as tricaprylin and triacetin and alcohols such as ethanol. The capsules can be formulated by filling a granule, a powder or a solution into a capsule made of gelatin, and the like.

Examples of the dosage form for a subcutaneous, intramuscular and intravenous administration include injections in the form of an aqueous or nonaqueous solution. In the aqueous solution, use is made of, for example, a physiological saline, and the like. In the nonaqueous solution, use is made of, for example, propylene glycol, polyethylene glycol, olive oil, ethyl oleate, and the like. If necessary, preservatives, stabilizers, etc., may be added thereto. The injections can be sterilized by a proper treatment, such as a filtration through the bacterial filter, or by an addition of a bacteriocide.

Examples of the dosage forms for a percutaneous administration include ointments and creams. The ointments and creams can be formulated by a conventional method through the use of fatty oils, such as castor oil and olive oil, petrolatums, etc., in the case of the ointments, and emulsifiers, such as diethylene glycol and sorbitan monofatty acid esters, etc., in the case of the creams.

Conventional suppositories, such as gelatin soft capsules, may be used for a rectal administration.

Although the dosage of the pyrrolo[2,3-d]pyrimidine derivative of the present invention varies depending upon the kind of disease, administration path, age and sex of patient, and severity of disease, etc., it is usually 1 to 500 mg/day/adult.

All of the compounds provided by the present invention (testing substances) have more than 2 g/kg (rat, P.O.) of LD₅₀.

### EXAMPLES

The present invention will now be described in more detail with reference to the following Examples.

### Reference Example: Synthesis of 2-allylamino-4-chloro-7-methyl-7H-pyrrolo[2,3-d]pyrimidine

### Procedure A:

A mixture of 5.00 g (29.6 mmol) of 2-amino-4-chloro-7H-pyrrolo[2,3-d]pyrimidine, 10.08 g (1.1 eq.) of p-anisylchlorodiphenylmethane, and 4.96 ml (12 eq.) of triethylamine in 65 ml of dimethylformamide (DMF) was stirred at room temperature for 30 min. After cooling to 0°C, 4.50 ml (2.44 eq.) of methyliodide and 3.00 g (2.53 eq.) of sodium hydride were added in order, and the mixture was stirred for one hour. And then, 5.36 ml (1.5 eq.) of allyl iodide and 2.00 (2.5 eq.) of sodium hydride were added to the reaction mixture followed by additional one hour stirring at 0°C. Finally 200 ml of 2N hydrochloric acid and 100 ml of diethyl ether were added and stirred for one hour at room temperature. The reaction mixture was neutralized with sodium bicarbonate and extracted with three portion of 100 ml of ethylacetate. The combined organic layer was washed with brine, dried over anhydrous magnesium sulfate. The solvents were removed in vacuo. The residual oily mixture was purified with silica-gel column chromatography (hexane: ethylacetate = 8:1 as elutant), to give 3.51 g (53.1 %) of 2-allylamino-4-chloro-7-methyl-7H-pyrrdo[2,3-d]pyrimidine.

### Procedure B:

To 300 ml of methylene chloride was added 26.9 g (159.5 mmol) of 2-amino-4-chloro-7H-pyrrolo[2,3-d] pyrimidine and 111 ml (5 eq.) of triethylamine, and the mixture was stirred at -30°C. Then 36.7 ml (1.1 eq.) of tert-butyldimethylsillyl trifluoromethane sulfonate was slowly dropwised to the mixture, and reacted for 1.5 hours. The crystals were completely dissolved to form a light brown solution. The mixture was allowed to worm to room temperature and filtrated with 200 g of silica-gel on a glass-filter. The filtrate and an elute eluted with 1 ℓ of methylene chloride was combined. After evaporation of the solution, to the oily residue was added 300 ml of aqueous 1N NaOH, and the aqueous solution was extracted with hexane (500 ml × 4). The combined organic layer was washed with water and brine in order, dried over anhydrous magnesium sulfate, and removed the solvents in vacuo. The resulting crystals were recrystallized from hexane, to give 35.27 g (yield 78.2%) of 2-tert-butyl-dimethylsilylamino-4-chloro-7H-pyrrolo[2,3-d]pyrimidine in the form of light brown plates (m.p. 114°C)
Physical Property:
¹H-NMR (CDCℓ₃) δ:
0.30(s, 6H), 0.98(s, 9H), 4.5(br-s, 1H)
6.4H(m, 1H) 6.9(m, 1H), 8.3(br-s, 1H)
Then, 44.0 g (115.6 mmol) of tert-butyldimethyl silylamino-4-chloro-7H-pyrrolo[2,3-d]pyrimidine and 13.5 ml (1.4 eq.) of methyl iodide in 150 ml of DMF, and 34.40 g (1.6 eq.) of potassium carbonate was reacted at room temperature for 15 hours with viguous stirring. After addition of water to the reaction mixture, the aqueous solution was extracted with hexane (200 ml × 4), washed with brine, and dried over anhydrous magnesium sulfate. The solvents were removed in vacuo, to give 45.87 g (154.5 mmol) (quantitative yield) of 2-ter-butyldimethylsilylamino-4-chloro-7-methyl-7H-pyrrolo[2,3-d]pyrimidine in the form of light yellow crystals. Under nitrogen atomphere the crystals and 21.19 ml (1.5 eq.) of allyl iodide were dissolved in 300 ml of DMF, and the reaction mixture was cooled to 0°C with vigorous stirring, then 9.27 g (1.5 eq.) of sodium hydride (60%), thoroughly washed by hexane, was added to that as hexane suspension.

The mixture was stirred for 10 min., and then 300 ml of water was slowly added to stop the reaction. The aqueous solution was extracted with hexane (300 ml × 4), the combined organic layer was washed with water and brine, and dried over anhydrous magnesium sulfate. After evaporation, 53.5 g of a yellow oily residue was given. The residue was dissolved in 30 ml of diethylether, and to the solution 50 ml of concentrated hydrochloric acid was added at 0°C with stirring, and the mixture was reacted for 10 min. After the reaction was completed, to the solution was added diethylether (100 ml × 2) to separate the organic layer. The aqueous layer was diluted with 200 ml of ice-water, and then neutralized with a 5N NaOH aqueous solution.

The Resulting precipitate was extracted with ethylacetate (250 ml × 3), the extract was washed with water and brine, dried over anhydrous magnesium sulfate, and evaporated, to give 33.57 g (yield 97.6%) of 2-allylamino-4-chloro-7-methyl-7H-pyrrolo[2,3-d]pyrimidine in the form of light yellow crystals.

The crystals were recrystallized from ethylalcohol to give 32.57 g (yield 94.0%) of the above-described compound as yellowish white plates (m.p. 113-114°C)
Physical Propaties:
¹H-NMR (CDCl₃), δ:
3.67(s, 3 H), 4.0-4.2(m, 2H), 3.9-5.4(m, 3H). 5.75-6.25(m, 1H), 6.34(d, 1H, J=3.5 Hz), 6.77(d, 1H, J=3.5 Hz).

| Elemental analysis: for C₁₀H₁₁N₄Cℓ, | | | |
|---|---|---|---|
| Calculated: | C, 53.94; | H, 4.98; | N, 25.16 |
| Found: | C, 53.90; | H, 4.98; | N, 25.11 |

### Example 1: Synthesis of (±)-2-allylamino-7-methyl-4-(1-phenylethyl-amino)-7H-pyrrolo[2,3-d]pyrimidine and its hydrochloride (156)

To 70 ml of n-butylalcohol was added 32.5 g (146.0 mmol) of 2-allylamino-4-chloro-7-methyl-7H-pyrrolo[2,3-d]pyrimidine prepared in Reference Example, 26.22 g (1.3 eq.) of potassium carbonate and 88 g (5.0 eq.) of (±)-phenyl-ethylamine, the mixture was stirred in an autoclave equipped with a stirrer at 165°C (5 atm) for 5 hours. After cooling to room temperature, 400 ml of water was added, and the mixture was extracted with ethylacetate (250 ml × 3). Combined organic layer was washed with water and brine, dried over anhydrous magnesium sulfate, and then the solvents and unreacted (±)-phenylethyl-amine were removed in vacuo. The red oily residue was crystallized with isopropylalcohol, to give 40.38 g (yield 90.0%) of (±)-2-allylamino-7-methyl-4-(1-phenylethyl-amino)-7H-pyrrolo[2,3-d]pyrimidine [(156) free base] in the form of crude crystals. They were recrystallized from isopropylalcohol to give 37.56 g (yield 83.7%) of colorless needles (m.p. 112-112.5°C). Then 30.00 g of the free base was dissolved in 800 ml of diethylether, and a saturated hydrochloric acid ethereal solution was added to form hydrochloride.

The diethylether and excess hydrochloric acid were evaporated and the resulting oily residue was crystallized with acetone.

The crystals were washed with acetone, and dried in vacuo at 80°C, to give 33.08 g (yield from the tree base, 98.6%) of (±)-2-allylamino-7-methyl-4-(1-phenylethylamino)-7H-pyrrolo[2,3-d]pyrimidine hydrochloride [(156) HCℓ · salt] in the form of colorless crystals (m.p. 167-170°C).
Physical Properties

### The Free Base

¹H-NMR (CDCℓ₃) δ:
1.60 (d, 3H, J=6,8), 3.60(s, 3H), 4.0(t, 2H, J=5.7), 4.65(br-t, 1H, J=5,7), 4.95(br-s, 1H), 4.9-5.4(m, 2H), 5.43(t, 1H, J=6.8), 5.7-6.2(m, 1H), 6.11(d, 1H, J=3.5), 6.54(d, 1H, J=3.5), 7.2-7.5(m, 5H)
IR(KBr) ν max, cm⁻¹
3240, 1620, 1560, 1450, 1285
UV(EtOH) λ max, nm
295, 228

| Elemental analysis: for C₁₅H₂₁N₅, | | | |
|---|---|---|---|
| Calculated: | C, 70.33; | H, 6.89; | N, 22.78. |
| Found: | C, 70.38; | H, 7.01; | N, 22.63. |

### The Hydrochloride

IR(KBr) ν max, cm⁻¹
3240, 1620, 1560, 1450, 1285
UV(EtOH) λ max, nm
296, 236, 207

| Elemental analysis: for C₁₈H₂₂N₅Cℓ, | | | | |
|---|---|---|---|---|
| Caluculated: | C, 62.87; | H, 6.45; | N, 20.37; | Cℓ, 10.26 |
| Found: | C, 62.84; | H, 6.40; | N, 20.23; | Cℓ, 10.29. |

In the following Examples, the compounds of the present invention were prepared by the procedures described in Example 1, using corresponding starting materials and reactants, respectively, as well as reaction solvents and coexisting bases indicated in the following tables, and each reaction was performed under the condition, i.e., reaction temperature, reaction times, and reaction vessel, indicated in said tables.

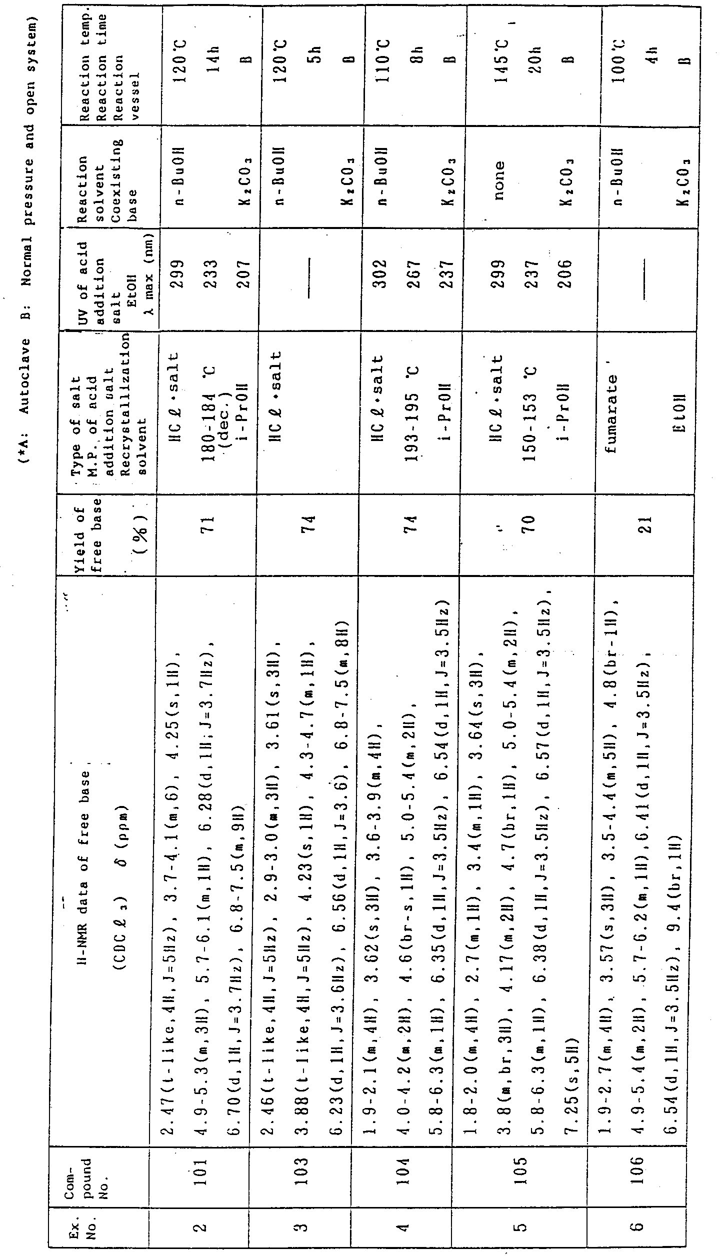

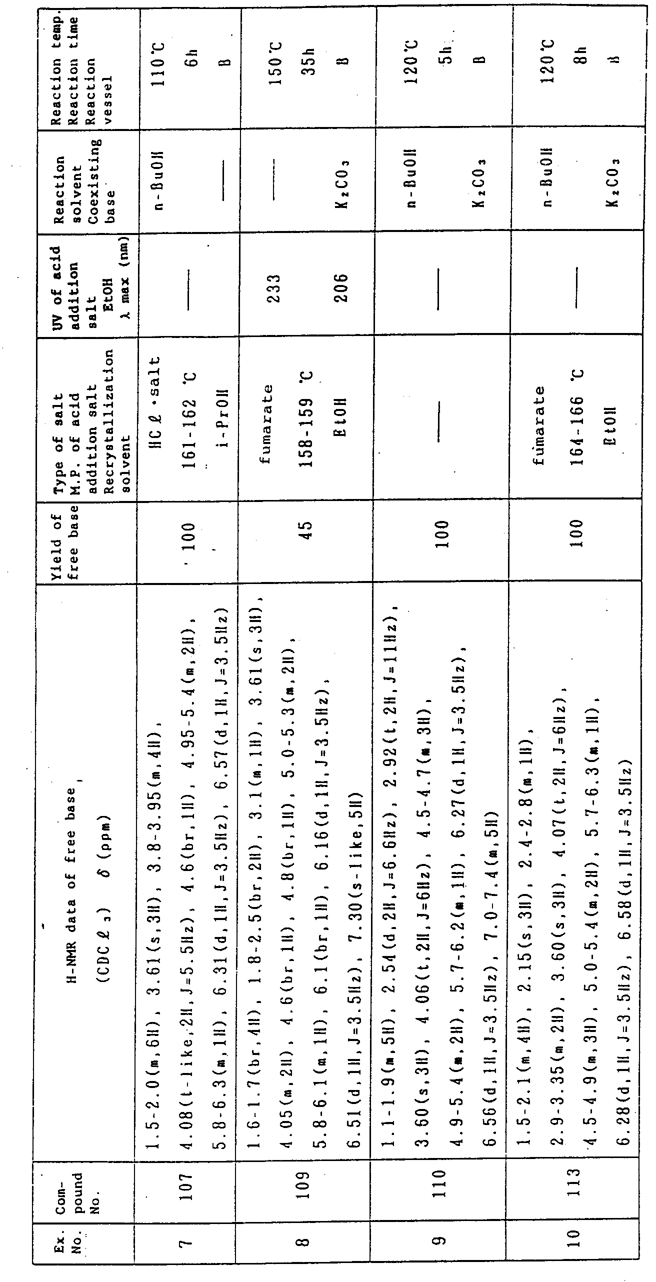

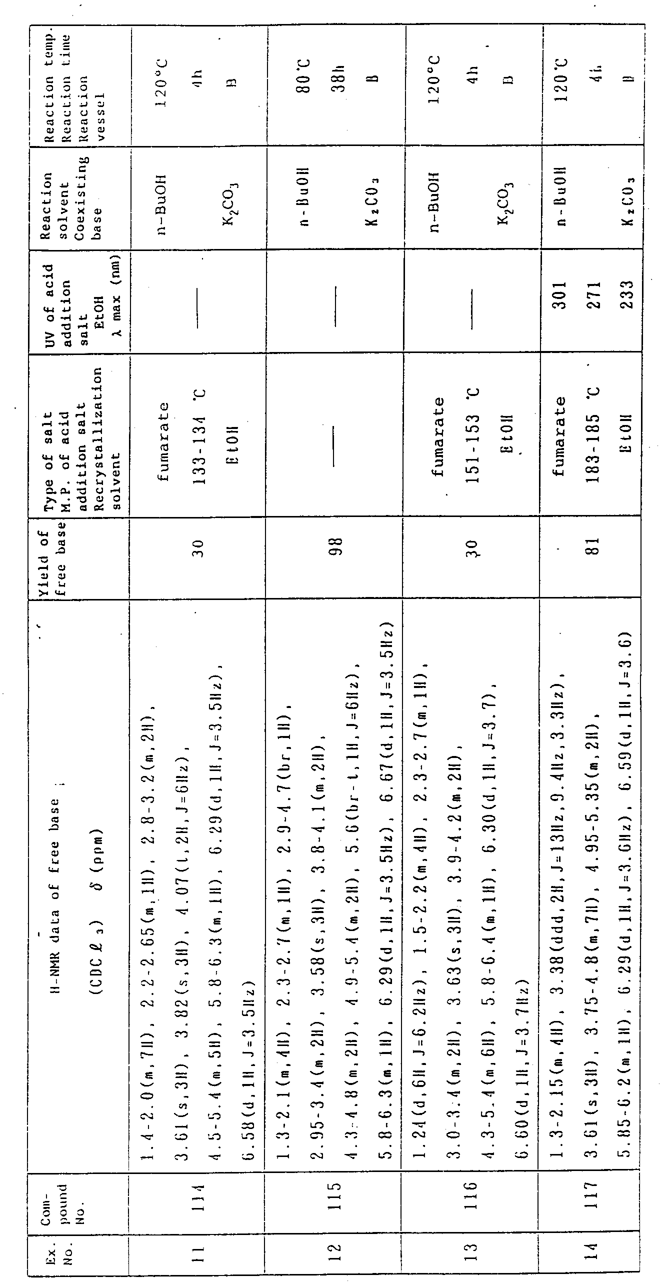

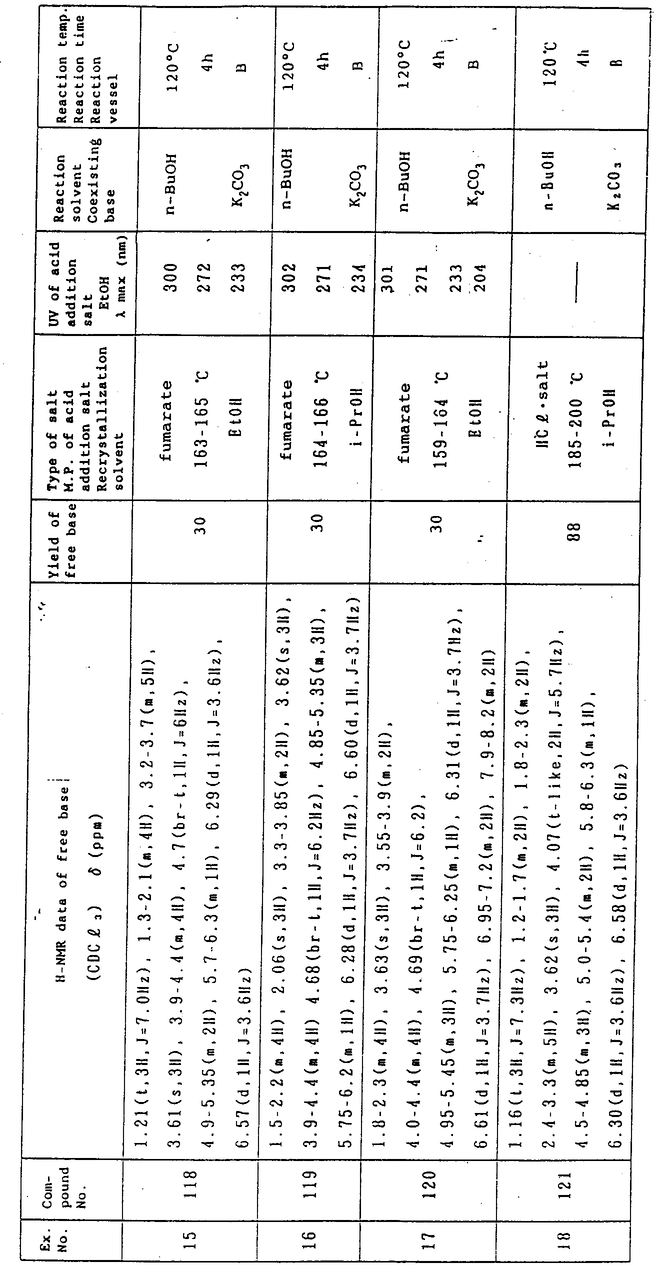

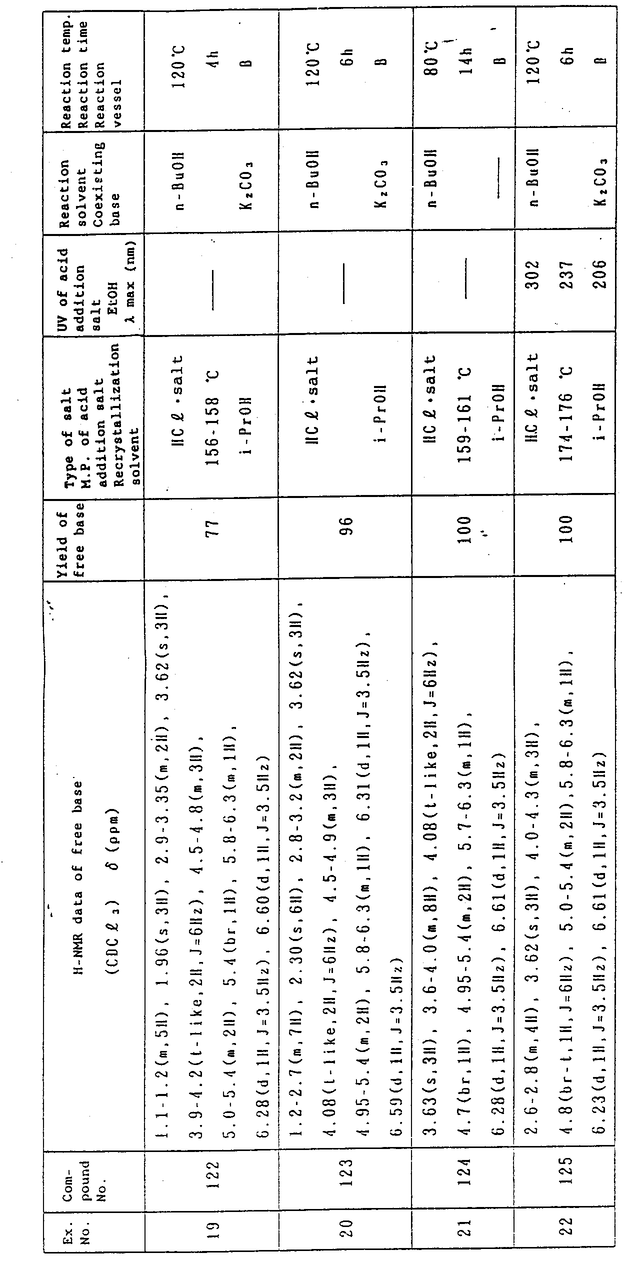

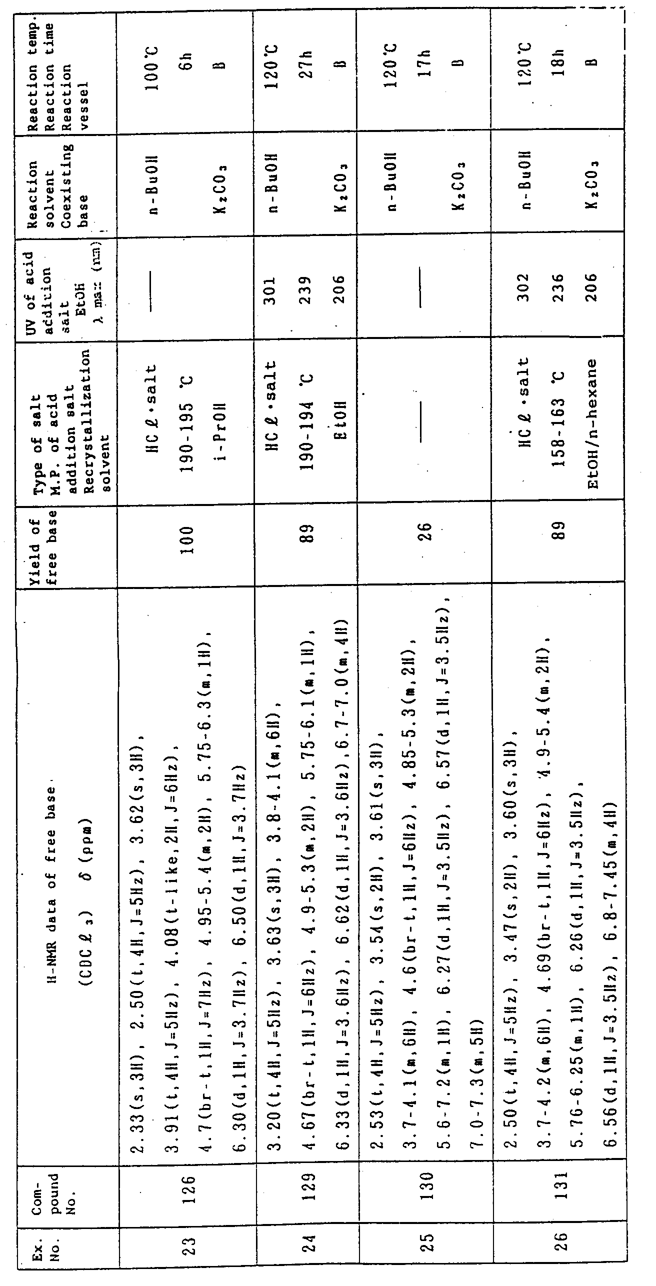

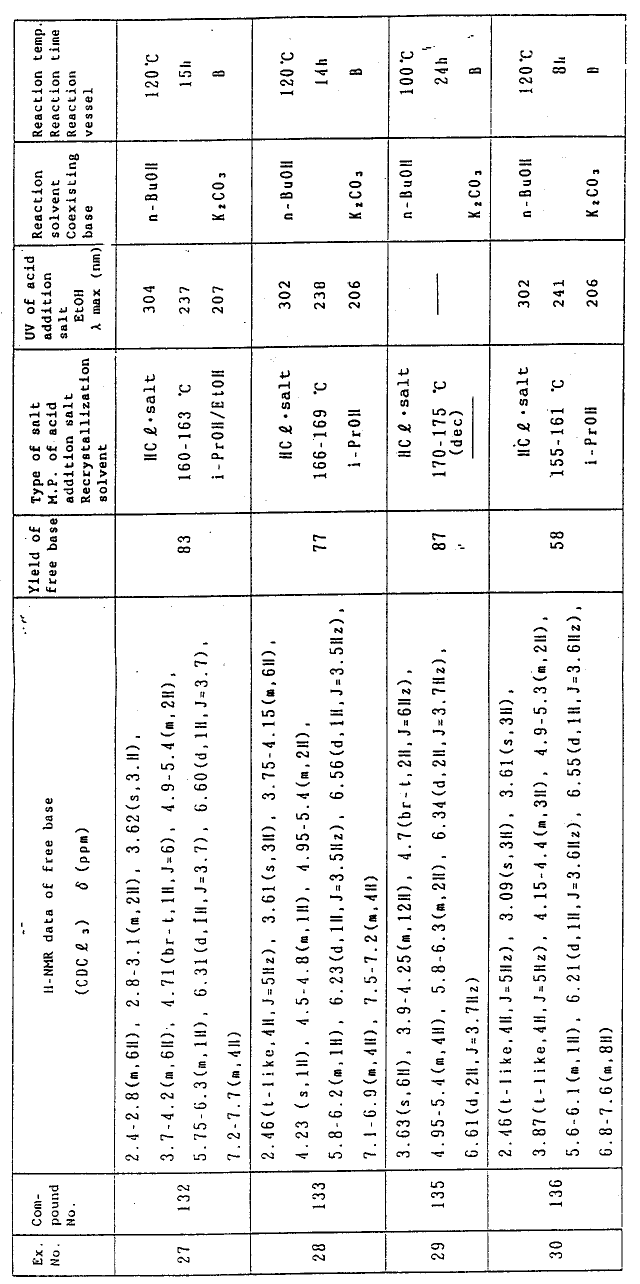

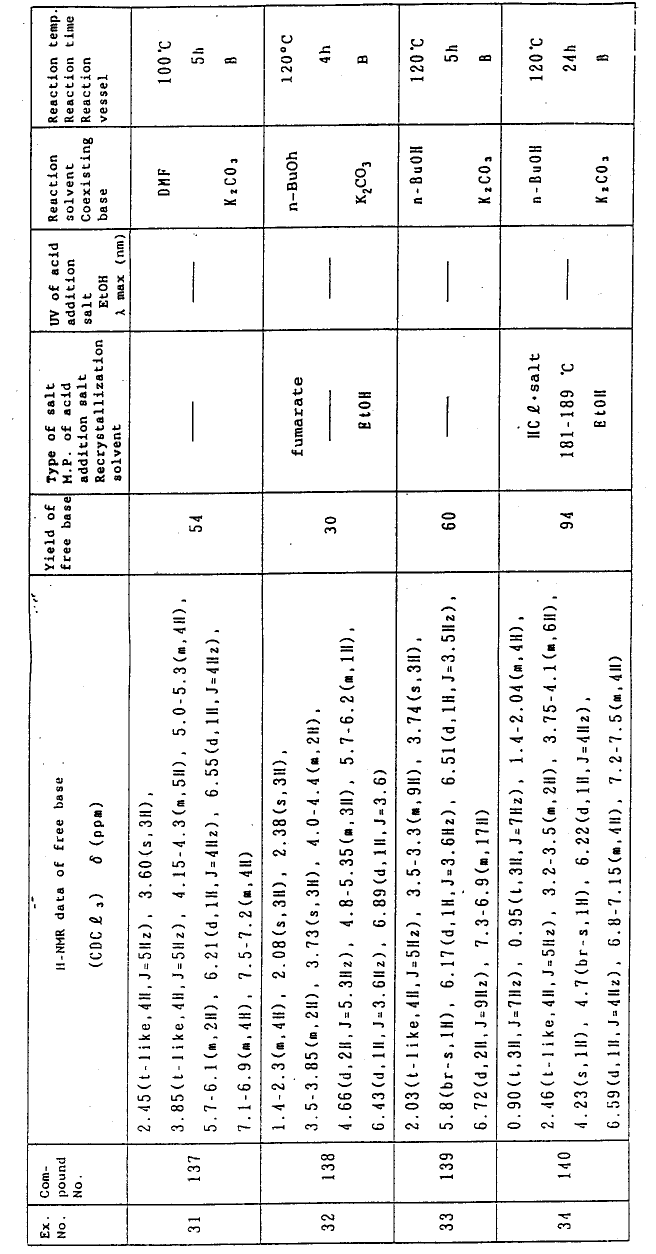

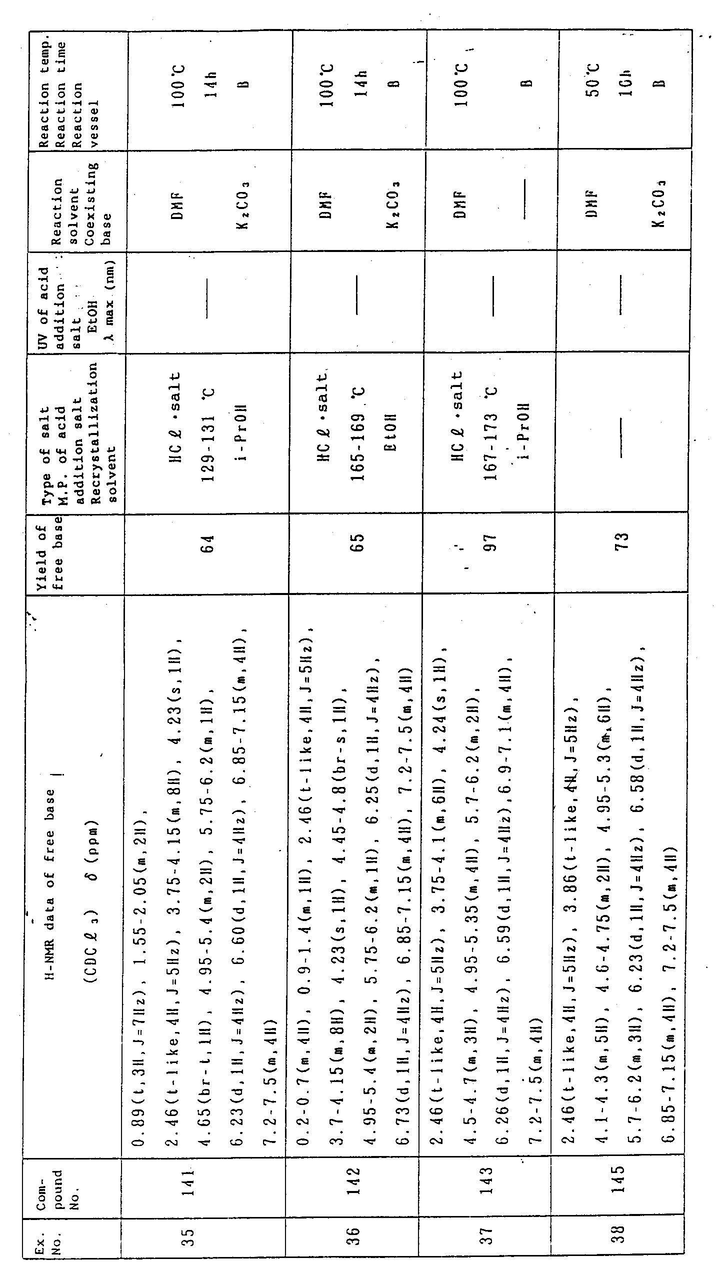

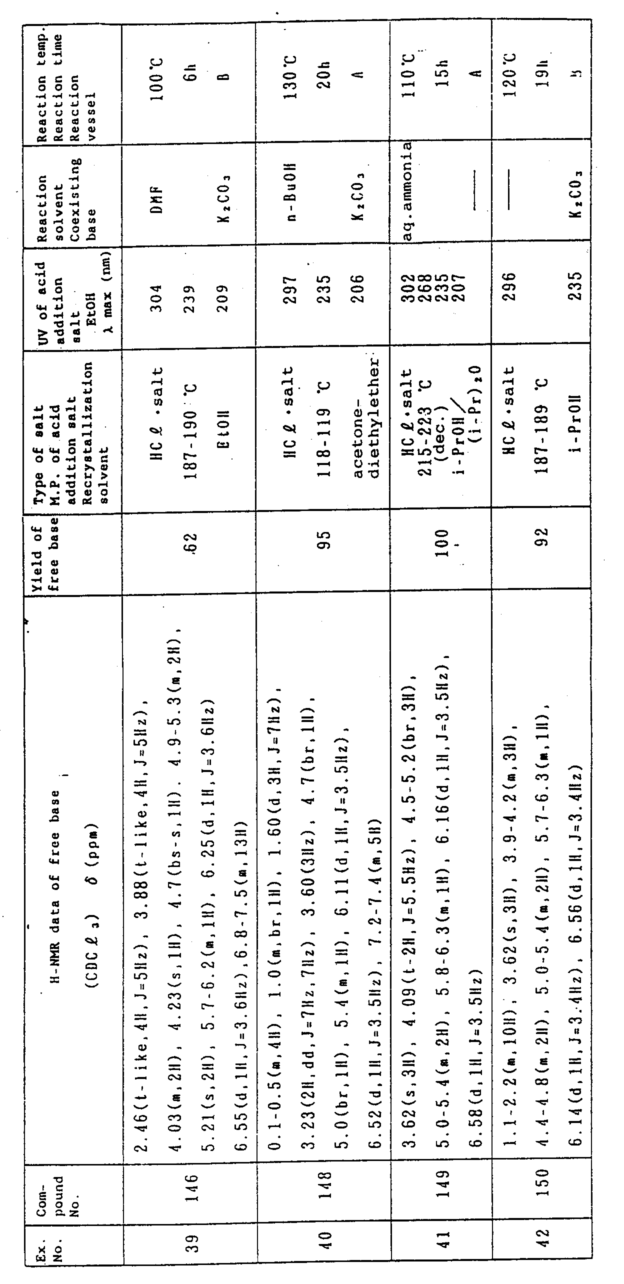

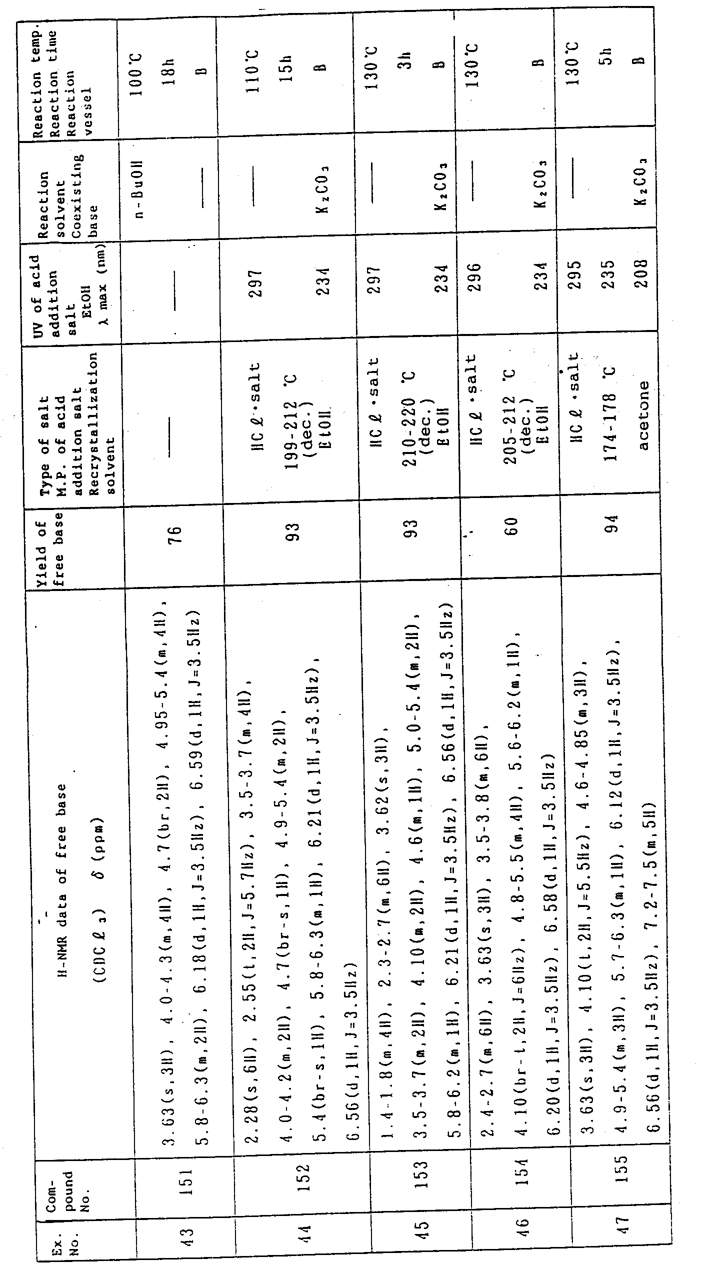

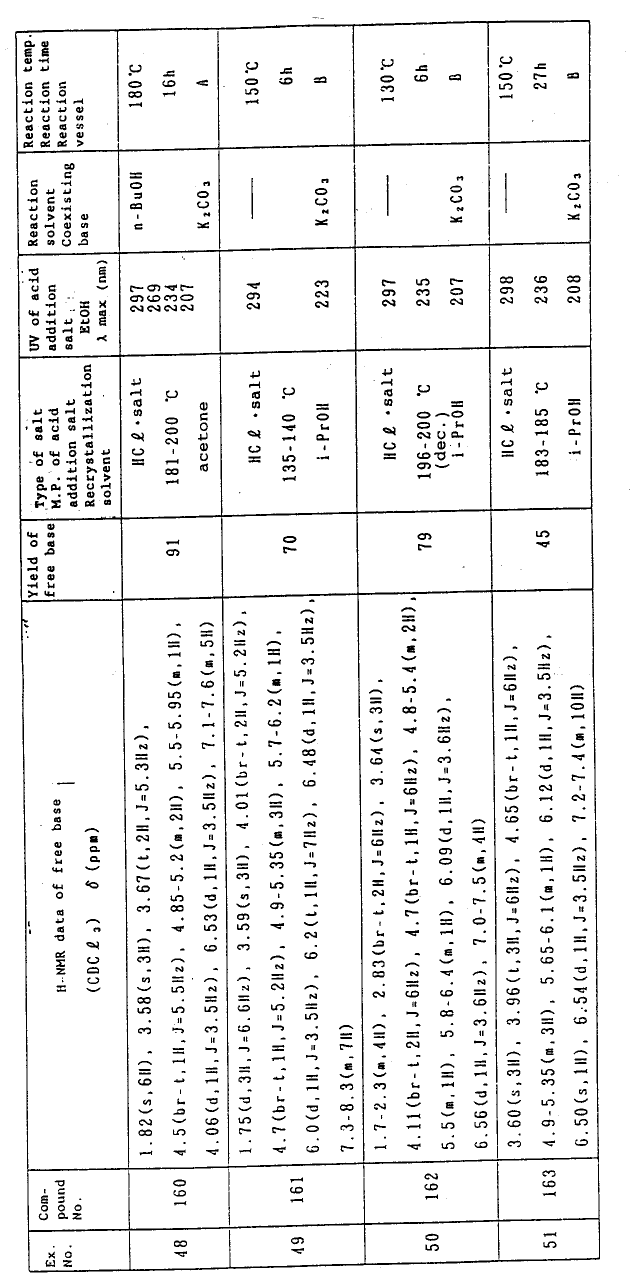

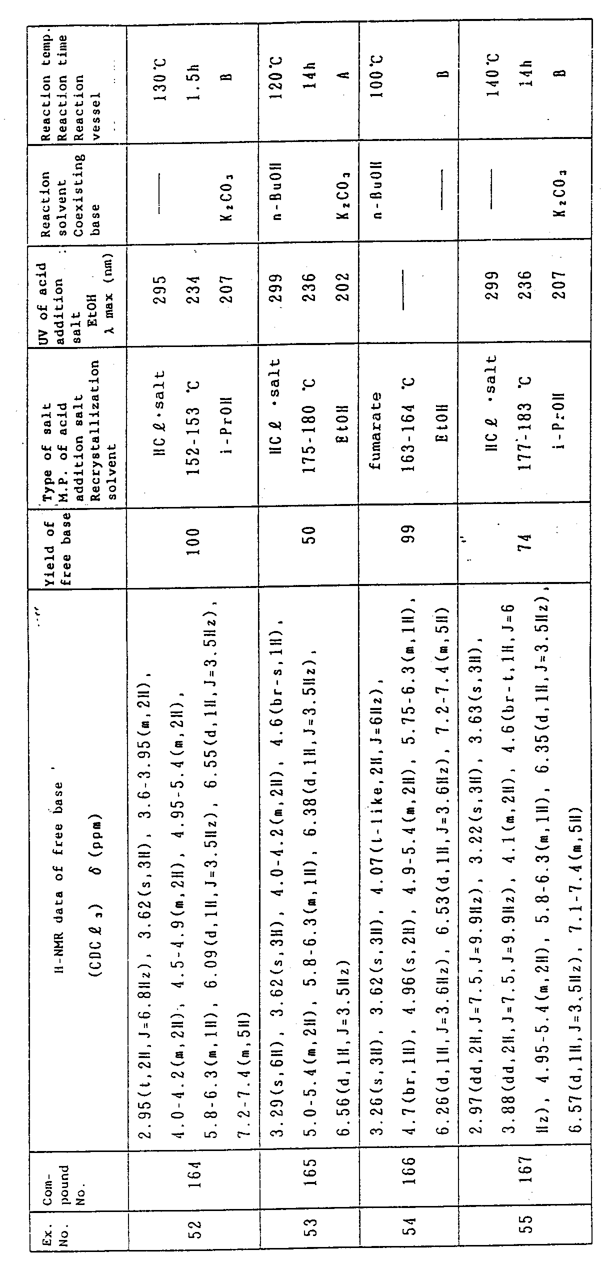

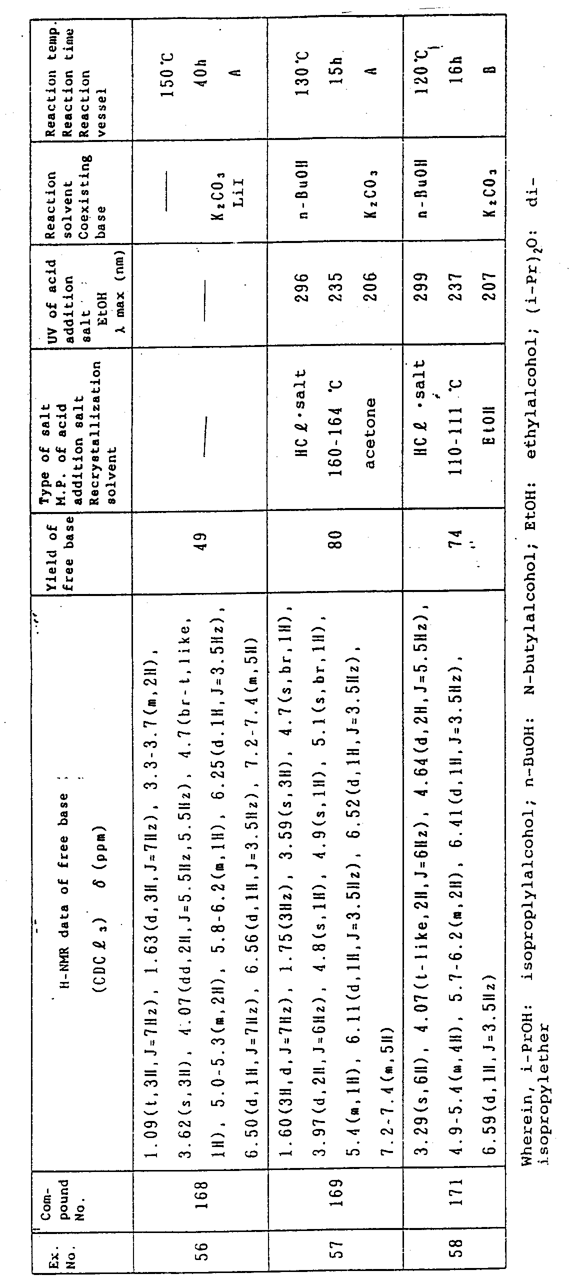

### Example 59: Effect on Partial Pressure Value of Gases in Arterial Blood (Injection system)

### 〈Method A〉

Male Wister strain rats (body weight about 300 g) were anesthetized intra-peritoneally with urethane, and a cannula was inserted into the respiratory tract and the femoral artery, respectively. A suspension (30-100 µm, 10 mg/ml) of carbon powder in a corn oil was intratrachealy injected to induce a hypoxemia state (PaO₂; 50-60 mmHg). A compound in accordance with the present invention was intravenously injected into these hypoxemia model animals by a continuous manner (0.1 mg/kg/min., 10 min), and then a partial pressure value of gases (PaO₂, PaCO₂) in arterial blood were immediately determined.

### 〈Method B〉

Male Wister strain rats (body about 300 g) were anesthetized with halothane inhalant, and then 2.0% acetic acid was intratrachealy injected at 0.6 ml/kg to induce a respiratory insufficiency. The animals were intra-peritoneally anesthetized with urethane-α-chloralose, and a cannula was inserted into the femoral artery. After the hypoxemic state (PaO₂: 60-70 mmHg) was observed a compound in accordance with the present invention (test substance) was intravenously injected into these hypoxemia model animals by a continuous manner (0.1 mg/kg/min), and then a partial pressure value of gases (PaO₂, PaCO₂) in arterial blood were immediately determined.

The results are as shown in Table 1 below.

**Table 1**

| Activity for Increasing PaO₂ and Decreasing PaCO₂ by Intravenous Injection | | | |
|---|---|---|---|
| Test Compound | Method | Activity for Increasing PaO₂ ΔPaO₂ | Activity for Decreasing PaCO₂ ΔPaCO₂ |
| 119 | A | +7.1 | -2.6 |
| 124 | B | +5.7 | -4.0 |
| 133 | A | +6.9 | -0.6 |
| 143 | A | +5.0 | +1.1 |
| 150 | B | +18.4 | -18.6 |
| 156 | B | +19.7 | -9.1 |
| 162 | B | +18.1 | -17.2 |
| 164 | B | +15.4 | -19.2 |
| 165 | B | +7.9 | +1.1 |
| 167 | B | +11.3 | -6.6 |
| Unit: mmHg (Indication of activity) ΔPaO₂ = (PaO₂ after administration - PaO₂ before administration) for test compound ΔPaCO₂ = (PaCO₂ after administration - PaCO₂ before administration) for test compound | | | |

### Example 60: Effect on Partial Pressure Value of Gases in Arterial Blood (Oral Administration System)

Male Wistar strain rats (body weight about 250 g) fasted overnight were anesthetized with halothane inhalant, and then a cannula was inserted into the femoral artery. After the animals recovered from the anesthesia, they were again anesthetized with halothane inhalant, and then 2.0% acetic acid was intratrachealy injected at 0.8 ml/kg to induce a hypoxemia state. After the hypoxemic state was observed over about 60 min., a compound in accordance with the present invention (test substance) was orally administered to the animals. On 60 min. after the administration, the partial pressure value of gases (PaO₂, PaCO₂) in arterial blood were determined.

The results are as shown in Table 2 below.

**Table 2**

| Activity for Increasing PaO₂ and Decreasing PaCO₂ by Intravenous Injection | | |
|---|---|---|
| Test Compound | Activity for Increasing PaO₂ ΔPaO₂ | Activity for Decreasing PaCO₂ ΔPaCO₂ |
| 124 | + | + |
| 156 | + + | + |
| 165 | + | ± |
| (Indication of activity) ΔPaO₂ = (PaO₂ after administration - PaO₂ before administration) for test compound ΔPaCO₂ = (PaCO₂ after administration - PaCO₂ before administration) for test compound ΔPaO₂ +: +3 - +6 mmHg ++: +6 - +9 mmHg ΔPaCO₂ ±: 0 - -3 mmHg +: -3 - -6 mmHg ++: -6 - -9 mmHg | | |

### Example 61: Preparation of Tablet

A tablet containing 30 mg of the compound prepared in Example 1 was prepared as follows:

| | |
|---|---|
| Compound prepared in Ex. 1 | 30 mg |
| Lactose | 87 mg |
| Starch | 30 mg |
| Magnesium stearate | 3 mg |

### Example 62: Preparation of Injection

A solution for injection containing 0.3 mg, based on 1 ml of the solution, of the compound prepared in Example 1 was prepared according to the following formulation.

| | |
|---|---|
| Compound prepared in Ex. 1 | 30 mg |
| Sodium chloride | 900 mg |
| Distilled water for injection | 100 ml |

### INDUSTRIAL APPLICABILITY

The compounds in accordance with the present invention, and pharmaceutical preparations thereof, are particularly useful for the treatment of hypoxemia associated with respiratory diseases, and further, an effective process for producing same is provided.

## Claims

1. A pyrrolo[2,3-d]pyrimidine derivative represented by the general formula [I]: 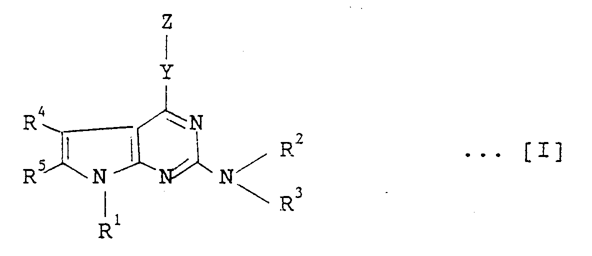 wherein
R¹ represents a hydrogen atom, or an unsubstituted or substituted alkyl, alkenyl or aralkyl group;
R² and R³ independently of each other, represent a hydrogen atom, or an unsubstituted or substituted alkyl, alkenyl, aralkyl or alkylcarbonyl group; or R² and R³ are optionally taken together with the adjacent nitrogen atom to form a cyclic amino group;
R⁴ and R⁵ independently of each other, represent a hydrogen atom, halogen atom, or an unsubstituted or substituted alkyl group;
Y is a linking group bonded to the pyrimidine ring via a nitrogen atom therein of the formula: 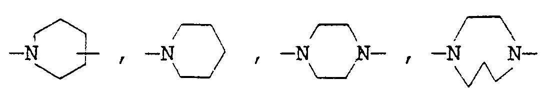 or -N(G)- , wherein G represents a hydrogen atom or an alkyl group;
Z represents a group bonded to a carbon or nitrogen atom in the linking group, and is a hydrogen atom, an unsubstituted or substituted alkyl, alkenyl, aralkyl, aryl, alkylcarbonyl, arylcarbonyl or arakylcarbonyl group; or represents a group bonded to a carbon atom in the linking group, and is a carboxyl, hydroxyl group; or an unsubstituted or substituted alkyloxycarbonyl, alkylcarbonyloxy, arylcarbonyloxy, aralkylcarbonyloxy, alkyloxy or alkyloxyiminoalkyl group, or a mono- or disubstituted alkyl- and/or alkylcarbonyl amino group; or Y and Z are taken together to form morpholino or thiomorpholino group;
each substituent in said substituted group is substituted at a chain or cyclic moiety of the alkyl, alkenyl, aralkyl or aryl moiety, respectively, and represents an alkyl, halogenated alkyl, alkylcarbonyl, alkyloxy, alkylcarbonyloxy, hydroxyl, mono- or dialkylamino, amino, morpholino, piperidino, nitro or cyano group, or a halogen atom;
and wherein the "alkyl" group herein contains 1 to 10 carbon atoms and "aryl" group means an aromatic hydrocarbon cyclic group or an aromatic heterocyclic group constructed of a mono-ring or a fused ring;
with a proviso that R² and R³ do not represent a hydrogen atom at the same time, and that when R¹ represents hydrogen atom, the combinations wherein one of R² and R³ represents a hydrogen atom and another represents an alkyl group are excluded; and
a pharmaceutically acceptable acid addition salt.

2. A pyrrolo[2,3-d]pyrimidine derivative and a pharmaceutically acceptable acid addition salt thereof in accordance with claim 1, wherein Y is a linking group having the formula:

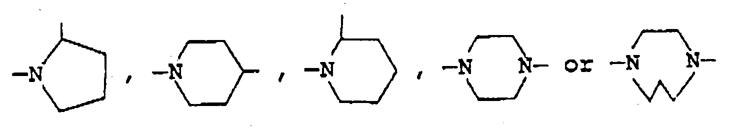

3. A pyrrolo[2,3-d]pyrimidine derivative and a pharmaceutically acceptable acid addition salt thereof in accordance with claim 1, wherein Y is a group -N(G)-, and G is a hydrogen atom, methyl group or ethyl group.

4. A pyrrolo[2,3-d]pyrimidine derivative and a pharmaceutically acceptable acid addition salt thereof in accordance with claim 1, wherein R¹ is a C₁ to C₆ alkyl group or allyl group.

5. A pyrrolo[2,3-d]pyrimidine derivative and a pharmaceutically acceptable acid addition salt thereof in accordance with claim 1, wherein R² is a hydrogen atom, R³ is an alkyl group, allyl group or 2-methylallyl group.

6. A pyrrolo[2,3-d]pyrimidine derivative and a pharmaceutically acceptable acid addition salt thereof in accordance with claim 1, wherein R⁴ and R⁵ are a hydrogen atom.

7. A pyrrolo[2,3-d]pyrimidine derivative and a pharmaceutically acceptable acid addition salt thereof in accordance with Claim 1, wherein R¹ is methyl group or allyl group; R² is a hydrogen atom; R³ is cyclopropylmethyl group; allyl group or 2-methylallyl group; R⁴ and R⁵ are a hydrogen atom; Y is a linking group having the formula: 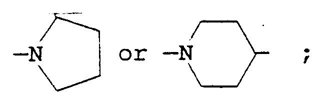 and Z is a hydrogen atom, alkyl group, alkylcarbonyl group, alkyloxycarbonyl group or alkylcarbonyloxy group; or Y is a linking group having the formula: 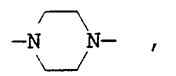 and Z is an alkyl group, or an unsubstituted or substituted aralkyl group; or Y is a linking group having the formula: -N(G)- , G is a hydrogen atom or alkyl group, and Z is cyclohexyl group, allyl group, an aralkyl group which may be substituted by one to three methyl groups on the alkyl chain; or Y and Z taken together is a morpholino group.

8. A pyrrolo[2,3-d]pyrimidine derivative and a pharmaceutically acceptable acid addition salt thereof in accordance with claim 1, wherein R¹ is a methyl group or allyl group; R² is a hydrogen atom; R³ is a cyclopropylmethyl group, allyl group or 2-methylallyl group; Y is a linking group having the formula: 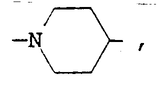 and Z is a hydrogen atom; or Y is a linking group having the formula: 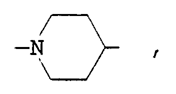 and Z is a hydrogen atom, isopropyloxycarbonyl group or acetyloxy group; or Y is a linking group having the formula: 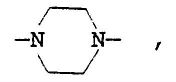 and Z is a methyl group or bis(4-fluorophenyl) methyl group; or Y is a linking group having the formula: -NH-, and Z is cyclohexyl group, allyl group, benzyl group, 1-phenylethyl group, 1-methyl-1-phenylethyl group, 2-phenylethyl group, 1-(1-naphthyl) ethyl group or 1,2,3,4-tetrahydronaphthalene-1-yl group; or Y is a linking group having the formula: -N(CH₃)-, and Z is methyl group, benzyl group or phenethyl group.

9. A process for producing a pyrrolo[2,3-d]pyrimidine derivative and a pharmaceutically acceptable acid addition salt in accordance with claim 1, characterized by reacting a halogenated pyrrolo[2,3-d]primidine derivative or an acid addition salt thereof having the general formula [II] 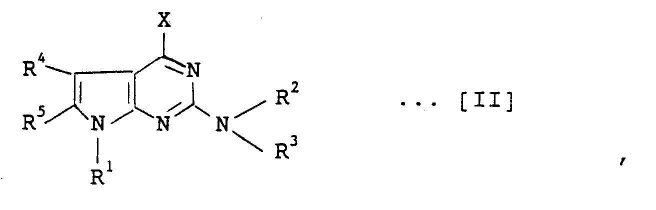 wherein R¹, R², R³, R⁴ and R⁵ have the same definitions as those of the formula [I] in claim 1, X is a halogen atom;
with an amine compound having the general formula [III]
Z - Y - H [III]
wherein Y and Z have the same definitions as those of said formula [I]; and further, mixing with an inorganic acid or organic acid, if necessary.

10. A pharmaceutical preparation comprising a pyrrolo[2,3-d]pyrimidine derivative in accordance with claim 1, as an efficacious ingredient.

11. A pharmaceutical preparation in accordance with claim 10, being efficacious for a treatment of hypoxemia.

## Patentansprüche

1. Pyrrolo-[2,3-d]-pyrimidin-Derivat entsprechend der allgemeinen Formel [I]: 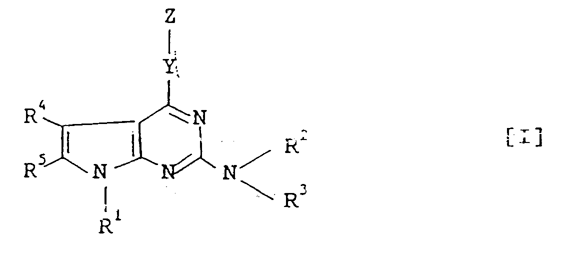 worin R¹ ein Wasserstoffatom oder eine unsubstituierte oder substituierte Alkyl-, Alkenyl- oder Aralkylgruppe repräsentiert;
worin R² und R³ unabhängig voneinander ein Wasserstoffatom oder eine unsubstituierte oder substituierte Alkyl-, Alkenyl- oder Aralkyl- oder Alkylcarbonylgruppe repräsentieren; oder worin R² und R³ gegebenenfalls zusammengenommen mit einem benachbarten Stickstoffatom eine cyclische Aminogruppe bilden;
worin R⁴ und R⁵ ein Wasserstoffatom, ein Halogenatom oder eine unsubstituierte oder substituierte Alkylgruppe repräsentieren;
worin Y eine mit einem Stickstoffatom des Pyrimidinrings verknüpfte Brückengruppe der Formel 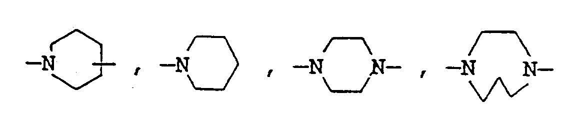 oder -N(G)- darstellt, worin G ein Wasserstoffatom oder eine Alkylgruppe repräsentiert;
worin Z eine an ein Kohlenstoffatom oder ein Stickstoffatom der Brückengruppe gebundene Gruppe ist und ein Wasserstoffatom oder eine unsubstituierte oder substituierte Alkyl-, Alkenyl-, Aralkyl-, Aryl-, Alkylcarbonyl-, Arylcarbonyl- oder Aralkylcarbonylgruppe repräsentiert; oder worin Z eine an ein Kohlenstoffatom der Brückengruppe gebundene Gruppe darstellt und eine Carboxyl- oder Hydroxylgruppe repräsentiert; oder worin Z eine unsubstituierte oder substituierte Alkyloxycarbonyl-, Alkylcarbonyloxy-, Arylcarbonyloxy-, Aralkylcarbonyloxy-, Alkyloxy- oder Alkyloxyiminoalkylgruppe oder eine mono- oder disubstituierte Alkyl- und/oder Alkylcarbonylaminogruppe repräsentiert; oder worin Y und Z zusammengenommen eine Morpholino- oder Thiomorpholinogruppe bilden;
worin jeder Substituent in der substituierten Gruppe an einem Ketten- oder einem cyclischen Teil der Alkyl-, Alkenyl-, Aralkyl- bzw. Arylgruppe substituiert ist und eine Alkyl-, halogenierte Alkyl-, Alkylcarbonyl-, Alkyloxy-, Alkylcarbonyloxy-, Hydroxyl-, Mono- oder Dialkylamino-, Amino-, Morpholino-, Piperidino-, Nitro- oder Cyanogruppe oder ein Halogenatom repräsentiert; und worin die 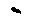 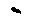 Alkyl'-gruppe hierin 1 bis 10 Kohlenstoffatome enthält und die 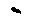 Aryl'-gruppe eine aromatische cyclische Kohlenwasserstoffgruppe oder eine aromatische heterocyclische Gruppe aus einem Monoring oder einem kondensierten Ring aufgebaut bedeutet;
unter der Bedingung, daß R² und R³ nicht gleichzeitig ein Wasserstoffatom repräsentieren, und daß, wenn R¹ ein Wasserstoffatom repräsentiert, die Kombinationen, bei denen einer der Reste R² und R³ ein Wasserstoffatom repräsentiert und ein anderer eine Alkylgruppe repräsentiert ausgeschlossen sind; und
ein pharmazeutisch geeignetes Säureadditionssalz.

2. Pyrrolo-[2,3-d]-pyrimidin-Derivat und ein pharmazeutisch geeignetes Säureadditionssalz hiervon gemäß Anspruch 1, worin Y eine Brückengruppe der Formel ist:

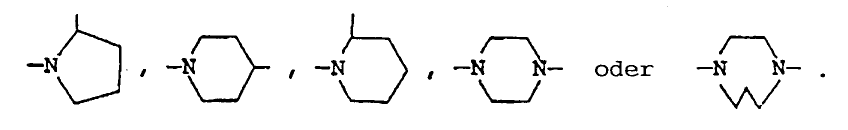

3. Pyrrolo-[2,3-d]-pyrimidin-Derivat und ein pharmazeutisch geeignetes Säureadditionssalz hiervon gemäß Anspruch 1, worin Y eine -N(G)- Gruppe ist und worin G ein Wasserstoffatom, eine Methylgruppe oder eine Ethylgruppe ist.

4. Pyrrolo-[2,3-d]-pyrimidin-Derivat und ein pharmazeutisch geeignetes Säureadditionssalz hiervon gemäß Anspruch 1, worin R¹ eine C₁-C₆-Alkylgruppe oder eine Allylgruppe ist.

5. Pyrrolo-[2,3-d]-pyrimidin-Derivat und ein pharmazeutisch geeignetes Säureadditionssalz hiervon gemaß Anspruch 1, worin R² ein Wasserstoffatom, R³ eine Alkylgruppe, Allylgruppe oder 2-Methylallylgruppe ist.

6. Pyrrolo-[2,3-d]-pyrimidin-Derivat und ein pharmazeutisch geeignetes Säureadditionssalz hiervon gemaß Anspruch 1, worin R⁴ und R⁵ ein Wasserstoffatom bedeuten.

7. Pyrrolo-[2,3-d]-pyrimidin-Derivat und ein pharmazeutisch geeignetes Säureadditionssalz hiervon gemaß Anspruch 1, worin R¹ eine Methylgruppe oder Allylgruppe ist; R² ein Wasserstoffatom ist; R³ eine Cyclopropylmethylgruppe, Allylgruppe oder 2-Methylallylgruppe ist; R⁴ und R⁵ ein Wasserstoffatom bedeuten; worin Y eine Brückengruppe mit der Formel ist: 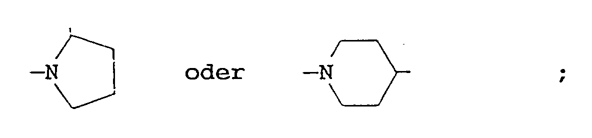 und worin Z ein Wasserstoffatom, eine Alkylgrupppe, Alkylcarbonylgruppe, Alkyloxycarbonylgruppe oder Alkylcarbonyloxygruppe bedeutet; oder worin Y eine Brückengruppe der Formel: 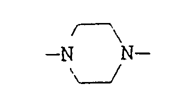 ist und Z eine Alkylgruppe oder eine unsubstituierte oder substituierte Aralkylgruppe ist; oder worin Y eine Brückengruppe der Formel -N(G)- ist, wobei G ein Wasserstoffatom oder eine Alkylgruppe ist,und Z eine Cyclohexylgruppe, eine Allylgruppe oder eine Aralkylgruppe ist, welche an der Alkylkette mit einer bis drei Methylgruppen substituiert sein kann; oder worin Y und Z zusammengenommen eine Morpholinogruppe bedeuten.

8. Pyrrolo-[2,3-d]-pyrimidin-Derivat und ein pharmazeutisch geeignetes Säureadditionssalz hiervon gemaß Anspruch 1, worin R¹ eine Methylgruppe oder Allylgruppe ist; worin R² ein Wasserstoffatom ist; worin R³ eine Cyclopropylmethylgruppe, eine Allylgruppe, oder eine 2-Methylallylgruppe ist; worin Y eine Brückengruppe der Formel 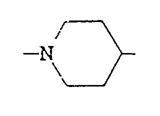 ist und Z ein Wasserstoffatom bedeutet; oder worin Y eine Brückengruppe der Formel 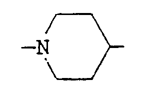 ist und Z ein Wasserstoffatom, eine Isopropyloxycarbonylgruppe oder Acetyloxygruppe bedeutet; oder worin Y eine Brückengruppe der Formel 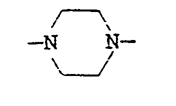 ist und Z eine Methylgruppe oder Bis-(4-fluorophenyl)methylgruppe bedeutet; oder worin Y eine Brückengruppe der Formel -NH- ist und Z eine Cyclohexylgruppe, eine Allylgruppe, eine Benzylgruppe, eine 1-Phenylethylgruppe, eine 1-Methyl-1-phenylethylgruppe, eine 2-Phenylethylgruppe, eine 1-(1-Naphthyl)-ethylgruppe oder eine 1,2,3,4-Tetrahydronaphthalin-1-ylgruppe bedeutet; oder worin Y eine Brückengruppe der Formel -N(CH₃)- ist und Z eine Methylgruppe, eine Benzylgruppe oder Phenethylgruppe bedeutet.

9. Verfahren zur Herstellung eines Pyrrolo-[2,3-d]-pyrimidin-Derivats und eines pharmazeutisch geeigneten Säureadditionssalzes hiervon gemäß Anspruch 1, gekennzeichnet durch die Umsetzung eines halogenierten Pyrrolo[2,3-d]-pyrimidin-Derivats oder eines pharmazeutisch geeigneten Säureadditionssalzes hiervon entsprechend der allgemeinen Formel [II] 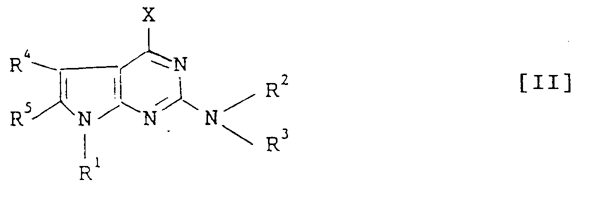 worin R¹, R², R³, R⁴ und R⁵ dieselbe Bedeutung wie jene in Formel [I] in Anspruch 1 aufweisen; worin X ein Halogenatom ist; mit einer Aminverbindung der allgemeinen Formel [III]
Z - Y - H [III]
worin Y und Z dieselbe Definition aufweisen wie in Formel [I]; und ferner, falls notwendig, Mischen mit einer anorganischen Säure oder einer organischen Säure.

10. Pharmazeutische Zubereitung, umfassend ein Pyrrolo-[2,3-d]-pyrimidin-Derivat gemäß Anspruch 1 als einen wirksamen Bestandteil.

11. Pharmazeutische Zubereitung nach Anspruch 10, welche bei einer Behandlung von Hypoxämie wirksam ist.

## Revendications

1. Dérivé de pyrrolo[2,3-d]pyrimidine représenté par la formule générale [I] : 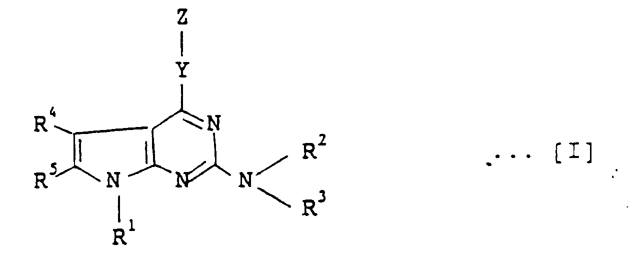 dans laquelle
R¹ représente un atome d'hydrogène ou un groupe alkyle, alcényle ou aralkyle substitué ou non substitué ;
R² et R³, indépendamment l'un de l'autre, représentent chacun un atome d'hydrogène ou un groupe alkyle, alcényle, aralkyle ou alkylcarbonyle non substitué ou substitué, ou encore R² et R³ sont éventuellement pris ensemble avec l'atome d'azote qui les relie pour former un groupe amino cyclique ;
R⁴ et R⁵, indépendamment l'un de l'autre, sont chacun un atome d'hydrogène, un atome d'halogène, ou un groupe alkyle substitué ou non substitué ;
Y est un groupe de liaison lié au noyau pyrimidine par l'intermédiaire d'un atome d'azote, selon la formule suivante : 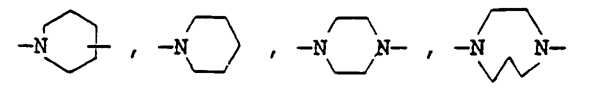 ou -N(G)- où G représente un atome d'hydrogène ou un groupe alkyle ;
Z représente un groupe lié à un atome de carbone ou d'azote du groupe de liaison et est un atome d'hydrogène, un groupe alkyle, alcényle, aralkyle, aryle, alkylcarbonyle, arylcarbonyle ou aralkylcarbonyle substitué ou non substitué ; ou encore représente un groupe lié à un atome de carbone du groupe de liaison, et est un groupe carboxyle ou hydroxyle ; ou encore un groupe alkyloxycarbonyle, alkylcarbonyloxy, arylcarbonyloxy, aralkylcarbonyloxy, alkyloxy ou alkyloxyiminoalkyle substitué ou non substitué, ou encore un groupe alkyl-et/ou alkylcarbonyl-amino mono- ou disubstitué ; ou encore Y et Z sont pris ensemble pour former un groupe morpholino ou thiomorpholino ;
chaque substituant dudit groupe substitué est substitué au niveau d'un fragment en chaîne ou d'un fragment cyclique du fragment respectivement alkyle, alcényle, aralkyle ou aryle, et représente un groupe alkyle, alkyle halogéné, alkylcarbonyle, alkyloxy, alkylcarbonyloxy, hydroxyle, mono- ou dialkylamino, amino, morpholino, pipéridino, nitro ou cyano, ou encore un atome d'halogène ;
et où le groupe 〈〈 alkyle 〉〉 contient de 1 à 10 atomes de carbone et le groupe 〈〈 aryle 〉〉 représente un groupe cyclique hydrocarboné aromatique ou un groupe hétérocyclique aromatique constitué d'un noyau unique ou d'un noyau condensé ;
du moment que R² et R³ ne représentent pas simultanément des atomes d'hydrogène, et que, quand R¹ est un atome d'hydrogène, sont exclues les combinaisons dans lesquelles l'un des radicaux R² et R³ représente un atome d'hydrogène, et l'autre représente un groupe alkyle; et
ses sels d'addition avec un acide, acceptables d'un point de vue pharmaceutique.

2. Dérivé de pyrrolo[2,3-d]pyrimidine et l'un de ses sels d'addition avec un acide, acceptable d'un point de vue pharmaceutique, selon la revendication 1, où Y est un groupe de liaison ayant la formule

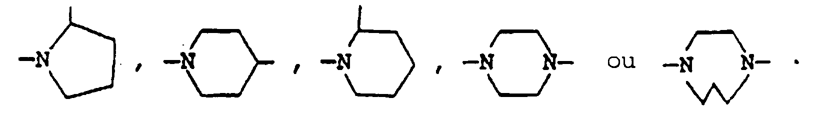

3. Dérivé de pyrrolo[2,3-d]pyrimidine et l'un de ses sels d'addition avec un acide, acceptable d'un point de vue pharmaceutique, selon la revendication 1, où Y est un groupe -N(G)-, et G est un atome d'hydrogène, un groupe méthyle ou un groupe éthyle.

4. Dérivé de pyrrolo[2,3-d]pyrimidine et l'un de ses sels d'addition avec un acide, acceptable d'un point de vue pharmaceutique, selon la revendication 1, où R¹ est un groupe alkyle en C₁ à C₆ ou un groupe allyle.

5. Dérivé de pyrrolo[2,3-d]pyrimidine et l'un de ses sels d'addition avec un acide, acceptable d'un point de vue pharmaceutique, selon la revendication 1, où R² est un atome d'hydrogène, R³ est un groupe alkyle, un groupe allyle ou un groupe 2-méthylallyle.

6. Dérivé de pyrrolo[2,3-d]pyrimidine et l'un de ses sels d'addition avec un acide, acceptable d'un point de vue pharmaceutique, selon la revendication 1, où R⁴ et R⁵ sont des atomes d'hydrogène.

7. Dérivé de pyrrolo[2,3-d]pyrimidine et l'un de ses sels d'addition avec un acide, acceptable d'un point de vue pharmaceutique, selon la revendication 1, où R¹ est le groupe méthyle ou le groupe allyle ; R² est un atome d'hydrogène ; R³ est un groupe cyclopropylméthyle ; un groupe allyle ou un groupe 2-méthylallyle ; R⁴ et R⁵ sont des atomes d'hydrogène ; Y est un groupe de liaison ayant la formule : 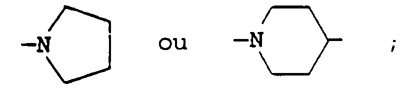 et Z est un atome d'hydrogène, un groupe alkyle, un groupe alkylcarbonyle, un groupe alkyloxycarbonyle ou un groupe alkylcarbonyloxy; ou encore Y est un groupe de liaison ayant la formule : 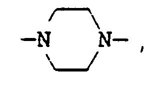 et Z est un groupe alkyle ou un groupe aralkyle substitué ou non substitué ; ou encore Y est un groupe de liaison ayant la formule -N(G)-, G est un atome d'hydrogène ou un groupe alkyle, et Z est le groupe cyclohexyle, un groupe allyle, un groupe aralkyle pouvant être substitué par 1 à 3 groupes méthyle sur la chaîne alkyle ; ou encore Y et Z, pris ensemble, représentent un groupe morpholino.

8. Dérivé de pyrrolo[2,3-d]pyrimidine et l'un de ses sels d'addition avec un acide, acceptable d'un point de vue pharmaceutique, selon la revendication 1, où R¹ est un groupe méthyle ou le groupe allyle ; R² est un atome d'hydrogène ; R³ est un groupe cyclopropylméthyle, le groupe allyle ou le groupe 2-méthylallyle ; Y est un groupe de liaison ayant la formule : 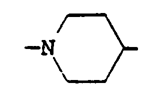 et Z est un atome d'hydrogène ; ou encore Y est un groupe de liaison ayant la formule : 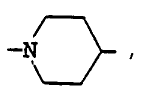 et Z est un atome d'hydrogène, le groupe isopropyloxycarbonyle ou le groupe acétyloxy ; ou encore Y est un groupe de liaison ayant la formule : 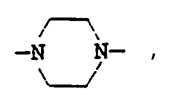 et Z est un groupe méthyle ou le groupe bis(4-fluorophényl)méthyle ; ou encore Y est un groupe de liaison ayant la formule -NH-, et Z est le groupe cyclohexyle, le groupe allyle, le groupe benzyle, le groupe 1-phényléthyle, le groupe 1-méthyl-1-phényléthyl, le groupe 2-phényléthyle, le groupe 1-(1-naphtyl)éthyle, ou le groupe 1,2,3,4-tétrahydronaphtalène-1-yle ; ou encore Y est un groupe de liaison ayant la formule -N(CH₃)-, et Z est le groupe méthyle, le groupe benzyle ou le groupe phénéthyle.

9. Procédé pour produire un dérivé de pyrrolo[2,3-d]pyrimidine et l'un de ses sels acceptable d'un point de vue pharmaceutique selon la revendication 1 caractérisé en ce qu'on fait réagir un dérivé halogéné de pyrrolo[2,3-d]pyrimidine ou l'un de ses sels d'addition avec un acide, ayant la formule générale [II] 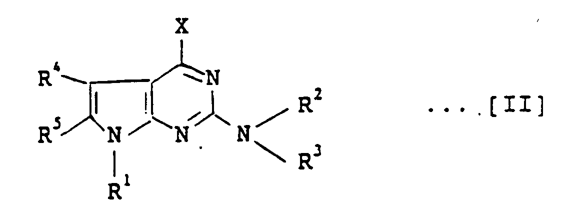 dans laquelle R¹, R², R³, R⁴ et R⁵ ont les significations données pour la formule [I] dans la revendication 1, et X est un atome d'halogène ;
avec une amine ayant la formule générale [III]
Z - Y - H [III]
où Y et Z sont tels que définis pour la formule [I] ; et de plus qu'on le mélange, si nécessaire, avec un acide minéral ou organique.

10. Préparation pharmaceutique comprenant un dérivé de pyrrolo[2,3-d]pyrimidine selon la revendication 1, comme principe actif.

11. Préparation pharmaceutique selon la revendication 10, qui est efficace dans le traitement de l'hypoxémie.
